(19) Europäisches Patentamt
European Patent Office
Office européen des brevets

(11) EP 2 670 807 B1

(12) **EUROPEAN PATENT SPECIFICATION**

(45) Date of publication and mention of the grant of the patent:
**26.04.2017 Bulletin 2017/17**

(21) Application number: **12709183.3**

(22) Date of filing: **03.02.2012**

(51) Int Cl.:
***C09B 63/00*** *(2006.01)* ***C09B 67/00*** *(2006.01)*
***A61K 8/04*** *(2006.01)*

(86) International application number:
**PCT/JP2012/053032**

(87) International publication number:
**WO 2012/105723 (09.08.2012 Gazette 2012/32)**

# (54) COMPOSITE PIGMENT AND METHOD FOR PREPARATION THEREOF

VERBUNDPIGMENT UND VERFAHREN ZU DEREN HERSTELLUNG

PIGMENT COMPOSITE ET PROCÉDÉ DE PRÉPARATION DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.02.2011 PCT/JP2011/052965**

(43) Date of publication of application:
**11.12.2013 Bulletin 2013/50**

(73) Proprietor: **L'Oréal**
**75008 Paris (FR)**

(72) Inventors:
- **MATSUFUJI, Shinichi**
**Kawasaki-shi**
**Kanagawa**
**213-0012 (JP)**
- **SHIMIZU, Momoko**
**Kawasaki-shi**
**Kanagawa**
**213-0012 (JP)**

(74) Representative: **Lavoix**
**Bayerstrasse 83**
**80335 München (DE)**

(56) References cited:
**EP-A2- 1 055 642** **EP-A2- 1 092 421**
**WO-A1-2010/098249** **WO-A1-2011/016143**
**DE-A1- 10 027 950** **DE-A1- 10 138 499**
**JP-A- 2007 254 429** **US-A- 5 512 094**

- **C.CONTADO ET AL.: "TiO2 in Commercial Sunscreen Lotion: Flow Field-flow Fractionation and ICP-AES together for Size Analysis", ANAL.CHEM., vol. 80, 2008, pages 7594-7608, XP002642392,**
- **BACSA R ET AL: "CVD synthesis of shape and size controlled ZnO nanoparticles for application as UV filters", ECS TRANSACTIONS, ELECTROCHEMICAL SOCIETY, vol. 25, no. 8, 1 January 2009 (2009-01-01), pages 1177-1183, XP009149325,**
- **DATABASE WPI Week 200774, Derwent Publications Ltd., London, GB; AN 2007-791710 & JP 2007 254429 A (KOSE KK) 04 October 2007**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 670 807 B1

Printed by Jouve, 75001 PARIS (FR)

## Description

### TECHNICAL FIELD

The present invention relates to a composite pigment comprising a small core particle which is at least partially covered by inorganic solid UV filter (s) particle and/or coloring pigment (s) (not claimed), as well as a method for preparing the composite pigment.

### BACKGROUND ART

Many cosmetics include one or more UV filters in order to shield UV rays. In particular, skin cosmetics commonly include inorganic solid UV filters such as fine particles of $TiO_2$ for protecting the skin from UV rays.

However, inorganic solid UV filters such as fine particles of $TiO_2$ can easily aggregate and have poor dispersibility. Therefore, it is often difficult to uniformly disperse them in the form of primary particles in cosmetics. Therefore, the UV filtering property of cosmetics including inorganic solid UV filters is difficult to be enhanced.

JP-A-2007-254429 discloses composite pigments comprising a relatively large core particle covered with inorganic solid UV filters.

The composite pigments based on a relatively large core particle covered with inorganic solid UV filters can provide improved UV filtering effects, because the aggregation of the inorganic solid UV filters can be prevented.

However, the UV filtering effects provided by the above composite pigments are still insufficient, and thus further improvement in the UV filtering effects is desired.

Further, coloring pigments can also easily aggregate and have poor dispersibility. Therefore, it is often difficult to uniformly disperse them in the form of primary particles in cosmetics. Therefore, the color of the cosmetics including coloring pigments can be opaque and dark.

Furthermore, there are some risks that fine particles of inorganic solid UV filter(s) and/or coloring pigment(s) may penetrate into the skin via pores on the skin, which may have adverse effects on the skin, because the barrier property of the skin is not strong in pores, and the inorganic solid UV filter(s) and/or coloring pigment(s) can irritate the skin when it or they contact the skin.

### DISCLOSURE OF INVENTION

Thus, an objective of the present invention is to provide a novel composite pigment which is based on inorganic solid UV filter(s) and which can provide better UV filtering effects.

Another objective of the present invention is to provide a novel composite pigment which is based on coloring pigment(s) and which can provide better coloring effects such as clear and bright color tone.

Another objective of the present invention is to reduce the risk of fine particles of inorganic solid UV filter(s) and/or coloring pigment(s) penetrating into the skin via pores on the skin, in order to reduce or prevent possible adverse effects on the skin by the inorganic solid UV filter(s) and/or coloring pigment(s). The present invention concerns a composite pigment comprising at least one small particle with a mean particle size of more than 100 nm and less than 1 $\mu$m, preferably less than 600 nm, and more preferably less than 400 nm,
wherein the surface of the small particle is at least in part covered with at least one layer comprising at least one inorganic solid UV filter particle. The inorganic solid UV filter may be selected from the group consisting of silicon carbide, metal oxides, and mixtures thereof.

In a preferred embodiment, the inorganic solid UV filter is a metal oxide.

The inorganic solid UV filter may have a mean particle size of 1 nm to 50 nm, preferably 5 nm to 40 nm, and more preferably 10 nm to 30 nm.

In a particular embodiment, the inorganic solid UV filter has at least one coating. The coating of the inorganic solid UV filter may comprise at least one compound selected from the group consisting of alumina, silica, aluminum hydroxide, silicones, silanes, fatty acids or salts thereof, fatty alcohols, lecithin, amino acids, polysaccharides, proteins, alkanolamines, waxes, (meth)acrylic polymers, organic UV filters, and (per)fluoro compounds.

The above at least one layer in the composite pigment according to the present invention may have a thickness of 1 nm to 50 nm, preferably 5 nm to 40 nm, and more preferably 10 nm to 30 nm.

The above at least one layer in the composite pigment according to the present invention may further comprise at least one additional UV filter, in particular at least one organic UV filter. The additional UV filter may be selected from the group consisting of solid benzotriazole derivatives, oxanilide derivatives, solid triazine derivatives, triazole derivatives, vinyl-group containing amides, cinnamic acid amides, sulfonated benzimidazoles; anthranilic derivatives, dibenzoylmethane derivatives, liquid cinnamic derivatives, salicylic derivatives, camphor derivatives, benzophenone derivatives, $\beta,\beta$-diphenylacrylate derivatives, liquid triazine derivatives, liquid benzotriazole derivatives, benzalmalonate derivatives,

benzimidazole derivatives, imidazoline derivatives, bis-benzoazolyl derivatives, p-aminobenzoic acid (PABA) and derivatives thereof, methylenebis(hydroxyphenylbenzotriazole) derivatives, benzoxazole derivatives, screening polymers and screening silicones, dimers derived from $\alpha$-alkylstyrene, 4,4-diarylbutadienes, and octocrylene and derivatives thereof, guaiazulene and derivatives thereof, rutin and derivatives thereof, flavonoids, biflavonoids, oryzanol and derivatives thereof, quinic acid and derivatives thereof, phenols, retinol, cysteine, aromatic amino acids, peptides having an aromatic amino acid residue, and mixtures thereof.

The small particle may comprise at least one inorganic material and/or at least one organic material, preferably at least one organic material.

The inorganic material may be selected from the group consisting of mica, synthetic mica, talc, sericite, boron nitride, glass flake, calcium carbonate, barium sulfate, titanium oxide, hydroxyapatite, silica, silicate, zinc oxide, magnesium sulfate, magnesium carbonate, magnesium trisilicate, aluminum oxide, aluminum silicate, calcium silicate, calcium phosphate, magnesium oxide, bismuth oxychloride, kaolin, hydrotalcite, mineral clay, synthetic clay, iron oxide, and mixtures thereof.

The organic material may be selected from the group consisting of poly(meth)acrylates, polyamides, silicones, polyurethanes, polyethylenes, polypropylenes, polystyrenes, polyhydroxyalkanoates, polycaprolactams, poly(butylene) succinates, polysaccharides, polypeptides, polyvinyl alcohols, polyvinyl resins, fluoropolymers, wax, amidosulfonic acid polyvalent metal salts, acylated amino acids, and mixtures thereof.

In a preferred embodiment, the small particle is an organic material. More preferably, the small particle is made from poly(meth)acrylates.

The weight ratio of the small particle(s) to the inorganic solid UV filter(s) may be 50:50 to 90:10, preferably 50:50 to 80:20, and more preferably 50:50 to 70:30.

The composite pigment according to the present invention can be prepared by a method comprising a step of subjecting:

at least one small particle with a mean particle size of more than 100 nm and less than 1 $\mu$m, preferably less than 600 nm, and more preferably less than 400 nm;
at least one inorganic solid UV filter and/or at least one coloring pigment; and
optionally at least one additional UV filter to a mechanochemical fusion process.

The coloring pigment may be selected from the group consisting of titanium dioxide, zirconium oxide, cerium oxide, zinc oxide, iron oxide, chromium oxide, manganese violet, ultramarine blue, chromium hydrate, ferric blue,-aluminum powder, copper powder, silver powder, gold powder, barium sulfate, carbon black, pigments of D&C type, lakes, pearlescent pigments, silica, and mixtures thereof. The present invention also relates to a composite pigment composition comprising
at least one composite pigment as explained above, and
at least one large particle with a mean particle size of 2 $\mu$m or more, preferably 3 $\mu$m or more, more preferably 4 $\mu$m or more, and even more preferably 5 $\mu$m or more,
wherein the surface of the large particle is optionally at least in part covered with at least one layer comprising at least one inorganic solid UV filter and/or at least one coloring pigment. The large particle may comprise at least one inorganic material and/or at least one organic material, preferably at least one organic material. Such inorganic and organic materials are disclosed above.

In a preferred embodiment, the large particle is made from polyamides.

The weight ratio of the small particle(s) to the large particle(s) may be 10:90 to 90:10, preferably 20:80 to 80:20, and more preferably 30:70 to 70:30.

The weight ratio of the small particle(s)/the large particle(s)/the inorganic solid UV filter(s) may be 9:81:10 to 27:3:70, preferably 8:72:20 to 45:5:50, and more preferably 7:63:30 to 63:7:30.

In a particular embodiment, the weight ratio of the small particle(s)/the large particle(s)/the inorganic solid UV filter(s) may be 20:50:30 to 50:20:30, preferably 35:15:50 to 15:35:50.

In a preferred embodiment, the weight ratio of the small particle(s)/the large particle(s)/the inorganic solid UV filter(s) may be 35:35:30.

In a preferred embodiment, the composite pigment composition comprises

- at least one composite pigment comprising at least one small particle made from poly(meth)acrylates with a mean particle size of more than 100 nm and less than 1 $\mu$m, preferably less than 600 nm, and more preferably less than 400 nm, wherein the surface of the small particle is at least in part covered with at least one layer comprising at least one inorganic solid UV filter, and - at least one large particle made from polyamides with a mean particle size of 2 $\mu$m or more, preferably 3 $\mu$m or more, more preferably 4 $\mu$m or more, and even more preferably 5 $\mu$m or more, wherein the surface of the large particle is optionally at least in part covered with at least one layer comprising at

least one inorganic solid UV filter.

The composite pigment composition according to the present invention can be prepared by a method comprising a step of subjecting:

a t least one small particle with a mean particle size more than 100 nm and of less than 1 μm, preferably less than 600 nm, and more preferably less than 400 nm;
a t least one inorganic solid UV filter and/or at least one coloring pigment;
a t least one large particle with a mean particle size of 2 μm or more, preferably 3 μm or more, more preferably 4 μm or more, and even more preferably 5 μm or more; and optionally at least one additional UV filter to a mechanochemical fusion process.

Another objective of the present invention is to provide a cosmetic composition with advantageous cosmetic and/or practical effects by using the composite pigment or composite pigment composition according to the present invention.
The above objective can be achieved by incorporating the composite pigment or composite pigment composition according to the present invention into a cosmetic composition.
Thus, the composite pigment or the composite pigment composition according to the present invention can be contained in a cosmetic composition, preferably a powdery cosmetic composition or a liquid cosmetic composition.

BRIEF DESCRIPTION OF DRAWINGS

Fig. 1 is a diagram showing how the matt effects were measured in Examples 41 and 42, as well as Reference 3.

Fig. 2 is a diagram showing how the haze effects were measured in Examples 41 and 42, as well as Reference 3.

Fig.3 is a SEM (Scanning Electron Microscopy) image of the small PMMA particle (Ex.18).

Fig. 4 is a SEM image of large Nylon particle (Ex.18).

Fig. 5 is TEM (Transmission Electron Microscopy) image of 1 composite pigment composition (Ex.18).

Fig. 6 is a representation of the composite pigment composition (ex. 18).

BEST MODE FOR CARRYING OUT THE INVENTION

After diligent research, the inventors have discovered that it is possible to obtain a new composite pigment providing enhanced UV filtering effects. The new composite pigment according to the present invention can also provide clear and bright color tone.
The new composite pigment according to the present invention comprises at least one small core particle with a mean particle size of less than 1 μm wherein the surface of the small core particle is at least in part covered with at least one layer comprising at least one inorganic solid UV filter and/or at least one coloring pigment.
Surprisingly, it was discovered that a single core particle with a smaller mean particle size of more than 100nm and less than 1 μm can provide better UV filtering and/or coloring effects than a single core particle with a larger mean particle size of 1 μm or more, and that the UV filtering and/or coloring effects by the small core particles can be further enhanced when being combined with large particles to form a composite pigment composition.
Since particles of inorganic solid UV filter(s) and/or coloring pigment(s) are firmly bonded on the core particle, the UV filter(s) and/or coloring pigments cannot penetrate into the skin via pores on the skin. In addition, even if inorganic solid UV filters and/or coloring pigment(s) irritate, a large amount of the inorganic UV filter(s) and/or coloring pigment(s) cannot directly contact with the skin, because they are present only on the core particle. Accordingly, the composite pigment or composite pigment composition according to the present invention is safer than the bulk of inorganic solid UV filters or coloring pigments.
Further, the composite pigment or composite pigment composition according to the present invention can provide a better feeling on use, because fine particles of inorganic solid UV filter(s) and/or coloring pigment(s) are firmly fixed on the core particle so that it is possible to reduce free fine particles which have a high friction coefficient such that they do not easily spread on the skin and provide an unpleasant feeling on use.

Furthermore, a cosmetic composition comprising the composite pigment or composite pigment composition according to the present invention can exert advantageous cosmetic and/or practical effects due to the inclusion of the composite pigment or composite pigment composition according to the present invention. For example, the cosmetic composition according to the present invention has better UV shielding effects. In addition, if the cosmetic composition is in the form of a powder, it also has a smooth feeling on use due to reduced friction, superior hiding effects for skin defects such as pores and fine lines, matt effects and good compactability such that it is difficult to chip away. On the other hand, if the cosmetic composition is in the form of a liquid, it also has good visual optical effects such as matt and haze effects.

Hereafter, each of the elements constituting the composite pigment and cosmetic composition according to the present invention will be described in a detailed manner.

(Small Core Particle)

The small core particle to be used for the composite pigment according to the present invention is not limited, as long as the small core particle has a mean particle size or a mean particle diameter of more than 100 nm and less than 1 μm, preferably less than 600 nm, and more preferably less than 400 nm.

The mean particle size or mean particle diameter here is an arithmetic mean diameter, and can be determined, for example, by calculating the average of the dimensions of one hundred particles chosen on an image obtained with a scanning electron microscope.

The small core particle can be in any shape. For example, it is possible to use a small core particle in the form of a plate with an aspect ratio of at least 5, preferably more than 10, more preferably more than 20, and more preferably more than 50. The aspect ratio can be determined by the average thickness and the average length according to the formula: aspect ratio = length/thickness.

If a plate-like particle is used for the present invention, it is preferable that the plate-like particle has a length ranging from more than 100 nm to less than 1 μm, preferably less than 600 nm, and more preferably less than 400 nm.

In a preferred embodiment, the small core particle has a spherical shape.

The material of the small core particle is not limited. The material can be at least one inorganic material and/or at least one organic material.

The inorganic material and/or organic material may be hollow or porous. The porosity of the material may be characterized by a specific surface area of from 0.05 $m^2/g$ to 1,500 $m^2/g$, more preferably from 0.1 $m^2/g$ to 1,000 $m^2/g$, and more preferably from 0.2 $m^2/g$ to 500 $m^2/g$ according to the BET method. However, it is preferable to use solid inorganic material(s) and/or solid organic material(s), preferably 'not hollow' materials.

Preferably, the inorganic material can be selected from the group consisting of mica, synthetic mica, talc, sericite, boron nitride, glass flakes, calcium carbonate, barium sulfate, titanium oxide, hydroxyapatite, silica, silicate, zinc oxide, magnesium sulfate, magnesium carbonate, magnesium trisilicate, aluminum oxide, aluminum silicate, calcium silicate, calcium phosphate, magnesium oxide, bismuth oxychloride, kaolin, hydrotalcite, mineral clay, synthetic clay, iron oxide, and mixtures thereof. In particular, natural mica, synthetic mica, sericite, kaolin, talc and mixtures thereof are preferable.

Preferably, the organic material can be selected from the group consisting of poly(meth)acrylates, polyamides, silicones, polyurethanes, polyethylenes, polypropylenes, polystyrenes, polyhydroxyalkanoates, polycaprolactams, poly(butylene) succinates, polysaccharides, polypeptides, polyvinyl alcohols, polyvinyl resins, fluoropolymers, waxes, amidosulfonic acid polyvalent metal salts, acylated amino acids, and mixtures thereof. As fluoropolymers, for example, PTFE may be used. As amidosulfonic acid polyvalent metal salts, for example, N-lauroyltaurine calcium may be used. As acylated amino acids, lauroyllysine may be used. Polyamides such as Nylon®, polyhydroxyalkanoates such as polylactic acids, poly(meth)acrylates such as polymethylmethacrylates, silicones, and mixtures thereof are more preferable.

In particular, polymethylmethacrylate particles such as MP-2200, MP-2701 and MP-1451 marketed by Soken in Japan are preferable as the small core particle.

The small core particle may or may not be coated beforehand.

In a particular embodiment, the small core particle is coated. The material of a coating of the small core particle is not limited, but an organic material such as an amino acid, an N-acylamino acid, an amido, a silicone and a modified silicone, is preferable. As the organic material, mention may be made of lauroyl lysine and acryl-modified silicone.

(Layer on Small Core Particle)

The small core particle is at least partially covered with at least one layer comprising at least one inorganic solid UV filter and/or at least one coloring pigment. The layer may be referred to as a coating layer. Preferably, 10% or more of the surface of the small core particle can be covered by the coating layer(s). More preferably, 50% or more of

the surface of the small core particle can be covered by the coating layer(s). More preferably, 80% or more of the small core particle can be covered by the coating layer(s). Most preferably, the entire surface of the small core particle can be covered by the coating layer(s).

The thickness of the coating layer may vary depending on several factors such as the size of the small core particle. Typically, the thickness of the coating layer may range from 1 nm to 50 nm, preferably 5 nm to 40 nm, and more preferably from 10 nm to 30 nm.

If there are two or more coating layers on the small core particle, the thickness and the composition of the coating layers may be the same as or different from each other.

The coating layer(s) may comprise, other than the inorganic solid UV filter(s) and/or the coloring pigment(s), any additional material(s) such as at least one additional UV filter. The additional material(s) may be present in an amount ranging from 1 to 50 wt% relative to the total weight of the additional material(s) and the inorganic solid UV filter(s) and/or the coloring pigment(s).

(Inorganic Solid UV Filter)

As described above, the coating layer(s) on the small core particle may comprise at least one inorganic solid UV filter. If two or more inorganic solid UV filters are used, they may be the same or different, preferably the same.

The inorganic solid UV filter used for the present invention may be active in the UV-A and/or UV-B region, preferably in the UV-B region or in the UV-A and UV-B region. It is preferable that the active UV filtering region of the inorganic solid UV filter and that of the additional UV filter are complementary to each other, in order to provide comprehensive UV protection. For example, it is preferable that the inorganic solid UV filter is active at least in the UV-B region and the additional UV filter is active at least in the UV-A region. The inorganic solid UV filter may be hydrophilic and/or lipophilic. The inorganic solid UV filter is properly insoluble in solvents such as water and ethanol commonly used in cosmetics. The term "solid" means solid at 25°C under 1 atm.

It is preferable that the inorganic solid UV filter is in the form of a fine particle such that the mean (primary) particle diameter thereof ranges from 1 nm to 50 nm, preferably 5 nm to 40 nm, and more preferably 10 nm to 30 nm. The mean (primary) particle size or mean (primary) particle diameter here is an arithmetic mean diameter.

The inorganic solid UV filter may be selected from the group consisting of silicon carbide, metal oxides which may or may not be coated, and mixtures thereof.

Preferably, the inorganic solid UV filters are selected from pigments (mean size of the primary particles: generally from 5 nm to 50 nm, preferably from 10 nm to 50 nm) formed of metal oxides, such as, for example, pigments formed of titanium oxide (amorphous or crystalline in the rutile and/or anatase form), iron oxide, zinc oxide, zirconium oxide or cerium oxide, which are all UV photoprotective agents well known per se. Preferably, the inorganic solid UV filters are selected from titanium oxide, zinc oxide, and more preferably titanium oxide.

The inorganic solid UV filter may or may not be coated. The inorganic solid UV filter may have at least one coating. The coating may comprise at least one compound selected from the group consisting of alumina, silica, aluminum hydroxide, silicones, silanes, fatty acids or salts thereof (such as sodium, potassium, zinc, iron or aluminum salts), fatty alcohols, lecithin, amino acids, polysaccharides, proteins, alkanolamines, waxes such as beeswax, (meth)acrylic polymers, organic UV filters, and (per)fluoro compounds.

It is preferable for the coating to include at least one organic UV filter. As the organic UV filter in the coating, a dibenzoylmethane derivative such as butyl methoxydibenzoylmethane (Avobenzone) and 2,2'-Methylenebis[6-(2H-Benzotriazol-2-yl)-4-(1,1,3,3-Tetramethyl-Butyl)Phenol] (Methylene Bis-Benzotriazolyl Tetramethylbutylphenol) marketed as "TINOSORB M" by BASF may be preferable.

In a known manner, the silicones in the coating(s) may be organosilicon polymers or oligomers comprising a linear or cyclic and branched or crosslinked structure, of variable molecular weight, obtained by polymerization and/or polycondensation of suitable functional silanes and essentially composed of a repetition of main units in which the silicon atoms are connected to one another via oxygen atoms (siloxane bond), optionally substituted hydrocarbon radicals being connected directly to the said silicon atoms via a carbon atom.

The term "silicones" also encompasses silanes necessary for their preparation, in particular alkylsilanes.

The silicones used for the coating(s) can preferably be selected from the group consisting of alkylsilanes, polydialkylsiloxanes and polyalkylhydrosiloxanes. More preferably still, the silicones are selected from the group consisting of octyltrimethylsilane, polydimethylsiloxanes and polymethylhydrosiloxanes.

Of course, the inorganic UV filters made of metal oxides may, before their treatment with silicones, have been treated with other surfacing agents, in particular with cerium oxide, alumina, silica, aluminum compounds, silicon compounds or their mixtures.

The coated inorganic solid UV filter may have been prepared by subjecting the inorganic solid UV filter to one or more surface treatments of a chemical, electronic, mechanochemical and/or mechanical nature with any of the compounds as described above, as well as polyethylenes, metal alkoxides (titanium or aluminum alkoxides), metal oxides,

sodium hexametaphosphate, and those shown, for example, in Cosmetics & Toiletries, February 1990, Vol. 105, pp. 53-64.

The coated inorganic solid UV filters may be titanium oxides coated:

with silica, such as the product "Sunveil" from Ikeda;
with silica and with iron oxide, such as the product "Sunveil F" from Ikeda;
with silica and with alumina, such as the products "Microtitanium Dioxide MT 500 SA" from Tayca, "Tioveil" from Tioxide, and "Mirasun TiW 60" from Rhodia;
with alumina, such as the products "Tipaque TTO-55 (B)" and "Tipaque TTO-55 (A)" from Ishihara, and "UVT 14/4" from Kemira; with alumina and with aluminum stearate, such as the product "Microtitanium Dioxide MT 100 T, MT 100 TX, MT 100 Z or MT-01" from Tayca, the products "Solaveil CT-10 W" and "Solaveil CT 100" from Uniqema, and the product "Eusolex T-AVO" from Merck;
with alumina and with aluminum laurate, such as the product "Microtitanium Dioxide MT 100 S" from Tayca;
with iron oxide and with iron stearate, such as the product "Microtitanium Dioxide MT 100 F" from Tayca;
with zinc oxide and with zinc stearate, such as the product "BR351" from Tayca;
with silica and with alumina and treated with a silicone, such as the products "Microtitanium Dioxide MT 600 SAS", "Microtitanium Dioxide MT 500 SAS" and "Microtitanium Dioxide MT 100 SAS" from Tayca;
with silica, with alumina and with aluminum stearate and treated with a silicone, such as the product "STT-30-DS" from Titan Kogyo;
with silica and treated with a silicone, such as the product "UV-Titan X 195" from Kemira;
with alumina and treated with a silicone, such as the products "Tipaque TTO-55 (S)" from Ishihara or "UV Titan M 262" from Kemira;
with triethanolamine, such as the product "STT-65-S" from Titan Kogyo;
with stearic acid, such as the product "Tipaque TTO-55 (C)" from Ishihara; or
with sodium hexametaphosphate, such as the product "Microtitanium Dioxide MT 150 W" from Tayca.

Other titanium oxide pigments treated with a silicone are preferably $TiO_2$ treated with octyltrimethylsilane and for which the mean size of the individual particles is from 25 and 40 nm, such as that marketed under the trademark "T 805" by Degussa Silices, $TiO_2$ treated with a polydimethylsiloxane and for which the mean size of the individual particles is 21 nm, such as that marketed under the trademark "70250 Cardre UF $TiO_2Si_3$" by Cardre, anatase/rutile $TiO_2$ treated with a polydimethylhydrosiloxane and for which the mean size of the individual particles is 25 nm, such as that marketed under the trademark "Microtitanium Dioxide USP Grade Hydrophobic" by Color Techniques.

Preferably, the following coated $TiO_2$ can be used as the coated inorganic UV filter:

Stearic acid (and) Aluminum Hydroxide (and) $TiO_2$, such as the product "MT-100 TV" from Tayca, with a mean primary particle diameter of 15 nm;
Dimethicone (and) Stearic Acid (and) Aluminum Hydroxide (and) $TiO_2$, such as the product "SA-TTO-S4" from Miyoshi Kasei, with a mean primary particle diameter of 15 nm;
Silica (and) $TiO_2$, such as the product "MT-100 WP" from Tayca, with a mean primary particle diameter of 15 nm;
Dimethicone (and) Silica (and) Aluminum Hydroxide (and) $TiO_2$, such as the product "MT-Y02" and "MT-Y-110 M3S" from Tayca, with a mean primary particle diameter of 10 nm;
Dimethicone (and) Aluminum Hydroxide (and) $TiO_2$, such as the product "SA-TTO-S3" from Miyoshi Kasei, with a mean primary particle diameter of 15 nm;
Dimethicone (and) Alumina (and) $TiO_2$, such as the product "UV TITAN M170" from Sachtleben, with a mean primary particle diameter of 15 nm, and
Silica (and) Aluminum Hydroxide (and) Alginic Acid (and) $TiO_2$, such as the product "MT-100 AQ" from Tayca, with a mean primary particle diameter of 15 nm.

In terms of UV filtering ability, $TiO_2$ coated with at least one organic UV filter is more preferable. For example, Avobenzone (and) Stearic Acid (and) Aluminum Hydroxide (and) $TiO_2$, such as the product "HXMT-100ZA" from Tayca, with a mean primary particle diameter of 15 nm, can be used.

The uncoated titanium oxide pigments are, for example, marketed by Tayca under the trademarks "Microtitanium Dioxide MT500B" or "Microtitanium Dioxide MT600B", by Degussa under the trademark "P 25", by Wacker under the trademark "Oxyde de titane transparent PW", by Miyoshi Kasei under the trademark "UFTR", by Tomen under the trademark "ITS" and by Tioxide under the trademark "Tioveil AQ".

The uncoated zinc oxide pigments are, for example:

those marketed under the trademark "Z-cote" by Sunsmart;

those marketed under the trademark "Nanox" by Elementis; and those marketed under the trademark "Nanogard WCD 2025" by Nanophase Technologies.

The coated zinc oxide pigments are, for example:

those marketed under the trademark "Oxide Zinc CS-5" by Toshiba (ZnO coated with polymethylhydrosiloxane);
those marketed under the trademark "Nanogard Zinc Oxide FN" by Nanophase Technologies (as a 40% dispersion in Finsolv TN, $C_{12}$-$C_{15}$ alkyl benzoate);
those marketed under the trademark "Daitopersion Zn-30" and "Daitopersion Zn-50" by Daito (dispersions in oxyethylenated polydimethylsiloxane/cyclopolymethylsiloxane comprising 30% or 50% of zinc nano-oxides coated with silica and polymethylhydrosiloxane);
those marketed under the trademark "NFD Ultrafine ZnO" by Daikin (ZnO coated with phosphate of perfluoroalkyl and a copolymer based on perfluoroalkylethyl as a dispersion in cyclopentasiloxane);
those marketed under the trademark "SPD-Z1" by Shin-Etsu (ZnO coated with a silicone-grafted acrylic polymer dispersed in cyclodimethylsiloxane);
those marketed under the trademark "Escalol Z100" by ISP (alumina-treated ZnO dispersed in an ethylhexyl methoxycinnamate/PVP-hexadecene copolymer/methicone mixture); and those marketed under the trademark "Fuji ZnO-SMS-10" by Fuji Pigment (ZnO coated with silica and polymethylsilsesquioxane); those marketed under the trademark "Nanox Gel TN" by Elementis (ZnO dispersed at 55% in $C_{12}$-$C_{15}$ alkyl benzoate with hydroxystearic acid polycondensate).

The uncoated cerium oxide pigments are marketed, for example, under the trademark "Colloidal Cerium Oxide" by Rhone-Poulenc.

The uncoated iron oxide pigments are, for example, marketed by Arnaud under the trademarks "Nanogard WCD 2002 (FE 45B)", "Nanogard Iron FE 45 BL AQ", "Nanogard FE 45R AQ" and "Nanogard WCD 2006 (FE 45R)", or by Mitsubishi under the trademark "TY-220".

The coated iron oxide pigments are, for example, marketed by Arnaud under the trademarks "Nanogard WCD 2008 (FE 45B FN)", "Nanogard WCD 2009 (FE 45B 556)", "Nanogard FE 45 BL 345" and "Nanogard FE 45 BL" or by BASF under the trademark "Oxyde de fer transparent".

Mention may also be made of mixtures of metal oxides, in particular of titanium dioxide and of cerium dioxide, including a mixture of equal weights of titanium dioxide coated with silica and of cerium dioxide coated with silica marketed by Ikeda under the trademark "Sunveil A", and also a mixture of titanium dioxide and of zinc dioxide coated with alumina, with silica and with silicone, such as the product "M 261" marketed by Kemira, or coated with alumina, with silica and with glycerol, such as the product "M 211" marketed by Kemira.

Coated inorganic solid UV filters are preferable, because the UV filtering effects of the inorganic solid UV filters can be enhanced. In addition, the coating(s) may function as a binder for fixing the UV filters on a small core particle.

If the inorganic solid UV filter(s) in the form of fine particles is/are used, the composite pigment according to the present invention has an effect that it can provide not a white appearance but a transparent or clear appearance, because the fine particles of the inorganic solid UV filters do not aggregate but spread on the core particle. It should be noted that free fine particles of inorganic solid UV filter(s) easily aggregate to give a white appearance to the skin.

The inorganic solid UV filter(s) may be used in the composite pigment according to the present invention in proportions such that the weight ratio of the small core particle to the inorganic solid UV filter(s) is 50:50 to 90:10, preferably 50:50 to 80:20, and more preferably 50:50 to 70:30.

(Coloring Pigment)

As described above, the coating layer(s) on the small core particle may comprise at least one coloring pigment (not claimed). The term "coloring pigment(s)" should be understood as meaning white or colored, inorganic or organic particle(s) of any shape which is/are insoluble and is/are intended to color a composition comprising them.

If coloring pigment(s) is/are used, the composite pigment according to the present invention has an effect in that it can provide a clearer appearance with high chroma, because the coloring pigments do not aggregate but spread on the substrate. It should be noted that free coloring pigments easily aggregate to give a dark appearance with low chroma to the skin.

The pigments can be white or colored, inorganic and/or organic and generally have a mean particle size greater or equal to 1$\mu$m.

Among the inorganic pigments that may be used, non-limiting mention may be made of titanium dioxide, optionally surface treated, zirconium or cerium oxide, as well as zinc, (black, yellow or red) iron or chromium oxide, manganese violet, ultramarine blue, chromium hydrate and ferric blue, barium sulfate, or metal powders, such as aluminum,

copper, silver or gold powder.

The particle size of the coloring pigment is not limited. In a particular embodiment, the coloring pigment may have a mean particle size of from 100 nm to less than 1 μm, preferably from 100 nm to less than 500 nm, and more preferably from 100 nm to less than 300 nm.

Among organic pigments that may be used, non-limiting mention may be made of carbon black, pigments of D&C type and lakes, such as lakes-based on cochineal carmine and on barium, strontium, calcium or aluminum. For example, Red 202 (Calcium bis[2-(3-carboxy-2-hydroxynephthylazo)-5-methylbenzenesulfonate) may be used as the pigment of D&C type.

Preferably, the coloring pigment is chosen from titanium dioxide, zirconium oxide, cerium oxide, zinc oxide, iron oxide, chromium oxide, manganese violet, ultramarine blue, chromium hydrate, ferric blue, aluminum powder, copper powder, silver powder, gold powder, barium sulfate, carbon black, pigments of D&C type, lakes, pearlescent pigments, and mixtures thereof.

The term "pearlescent pigments" should be understood as meaning iridescent particles of any shape, such as particles produced by certain shellfish in their shells or else synthesized.

The pearlescent agents can be chosen from white pearlescent agents, such as mica covered with titanium dioxide or with bismuth oxychloride; colored pearlescent agents, such as titanium oxide-coated mica covered with iron oxide, titanium oxide-coated mica covered with ferric blue or chromium oxide, or titanium oxide-coated mica covered with an organic pigment of the abovementioned type; and pearlescent agents based on bismuth oxychloride.

The coloring pigment(s) may be used in the composite pigment according to the present invention in proportions such that the weight ratio of the small core particle to the coloring pigment(s) is 50:50 to 90:10, preferably 50:50 to 80:20, and more preferably 50:50 to 70:30.

(Additional UV Filter)

As described above, the coating layer on the small core particle may further comprise at least one additional UV filter. If two or more additional UV filters are used, they may be the same or different, preferably the same.

The additional UV filter used for the present invention may be active in the UV-A and/or UV-B region, preferably in the UV-A region or in the UV-A and UV-B region. The additional UV filter may be hydrophilic and/or lipophilic.

The additional UV filter may be solid or liquid. The terms "solid" and "liquid" mean solid and liquid, respectively, at 25°C under 1 atm. The additional UV filter may be made from at least one organic or inorganic material, preferably at least one organic material.

If organic solid UV filter(s) in the form of fine particles is/are used, as the additional UV filter(s), it is preferable that the organic solid UV filter is in the form of a fine particle such that the mean (primary) particle diameter thereof ranges from 1 nm to 50 nm, preferably 5 nm to 40 nm, and more preferably 10 nm to 30 nm.

If organic solid UV filter(s) in the form of fine particles is/are used, the composite pigment according to the present invention has an effect that it can provide a transparent or clear appearance, because the fine particles of the organic solid UV filter(s) do not aggregate but spread on the core particle.

It should be noted that free fine particles of organic solid UV filter(s) can easily aggregate.

Further, if organic solid UV filter(s) in the form of fine particles is/are used, the composite pigment according to the present invention has an additional effect that the particles of the inorganic solid UV filter(s) can be well dispersed in the coating layer due to the presence of the organic solid UV filter(s), and therefore, the inorganic solid UV filter(s) can be present in the coating layer in the form of primary particles. On the other hand, in the above case, the particles of the organic solid UV filter(s) can also be well dispersed in the coating layer due to the presence of the inorganic solid UV filter(s), and therefore, the organic solid UV filter(s) can be present in the coating layer in the form of primary particles. Accordingly, the UV filtering effects by the inorganic solid UV filter(s) as well as the organic solid UV filter(s) can be enhanced together.

The material of the organic solid UV filter(s) is not limited as long as it is organic. If two or more organic solid UV filters are used, the material(s) of the organic solid UV filters may be the same as or different from each other.

The organic solid UV filter may be selected from the group consisting of benzotriazole derivatives, oxanilide derivatives, triazine derivatives, triazole derivatives, vinyl-group containing amides, cinnamic acid amides, and sulfonated benzimidazoles.

A preferred class of solid oxanilide UV absorbers is that having the formula:

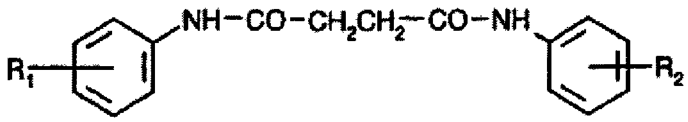 (1)

in which $R_1$ and $R_2$, independently, are $C_1$-$C_{18}$ alkyl or $C_1$-$C_{18}$ alkoxy. A preferred compound of formula (1) is N-(2-ethoxyphenyl)-N'-(2-ethylphenyl)-ethanediamide.

A preferred class of solid triazine UV absorbers is that having the formula:

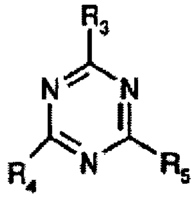

(2)

in which $R_3$, $R_4$ and $R_5$, independently, are H, OH, $C_1$-$C_{18}$ alkoxy, $NH_2$, NH-$R_6$ or $N(R_6)_2$ in which $R_6$ is $C_1$-$C_{18}$ alkyl, $OR_6$ in which $R_6$ is $C_1$-$C_{18}$ alkyl, phenyl, phenoxy or anilino, or pyrrole, in which the respective phenyl, phenoxy or anilino, or pyrrolo moieties are optionally substituted by one, two or three substituents selected from OH, carboxy, CO-$NH_2$, $C_1$-$C_{18}$ alkyl or alkoxy, $C_1$-$C_{18}$ carboxyalkyl, $C_5$-$C_8$ cycloalkyl, a methylidenecamphor group, the group $-(CH=CH)_mC(=O)-OR_6$ in which m is 0 or 1 and $R_6$ has the same meaning above, or the group

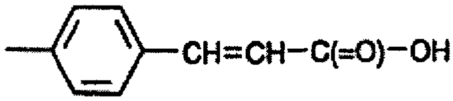

or the corresponding alkali metal, ammonium, mono-, di- or tri-$C_1$-$C_4$ alkylammonium, mono-, di- or tri-$C_2$-$C_4$ alkanolammonium salts, or the $C_1$-$C_{18}$ alkyl esters thereof.

Preferred compounds of formula (2) are those having one of the formulae:

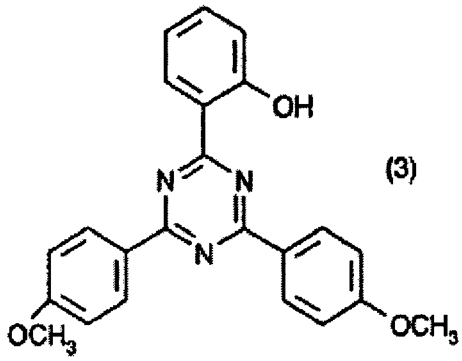

(3)

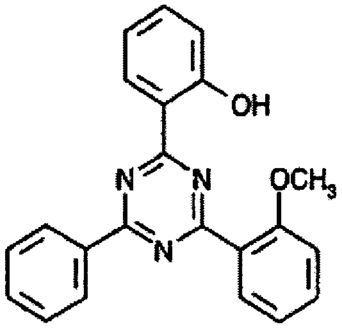

(4)

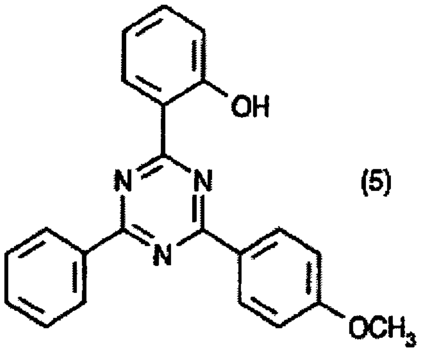

(5)

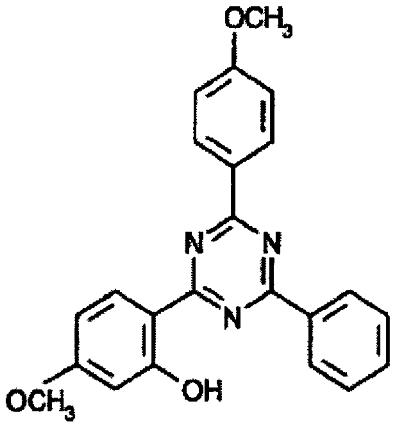

(6)

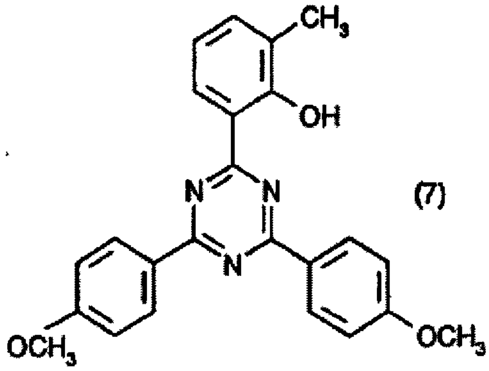

(7)

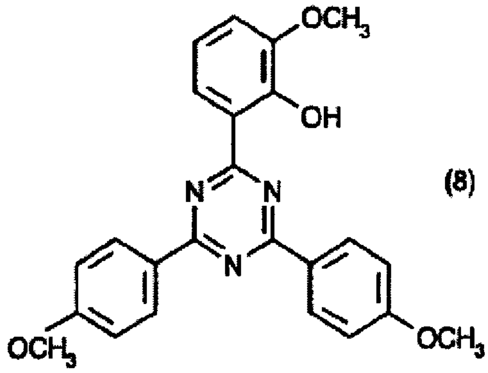

(8)

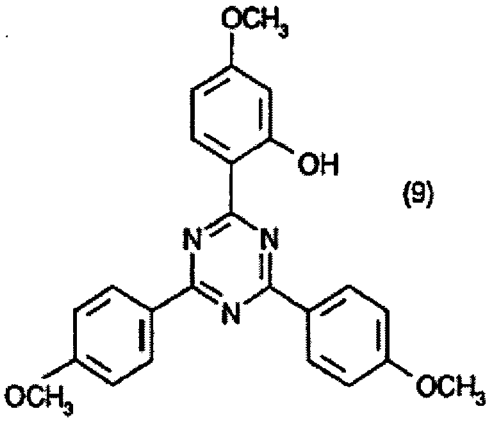

(9)

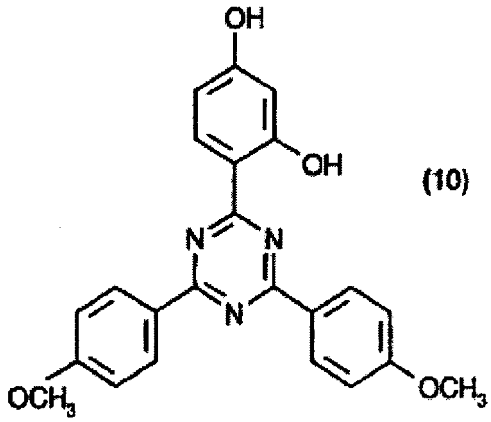

(10)

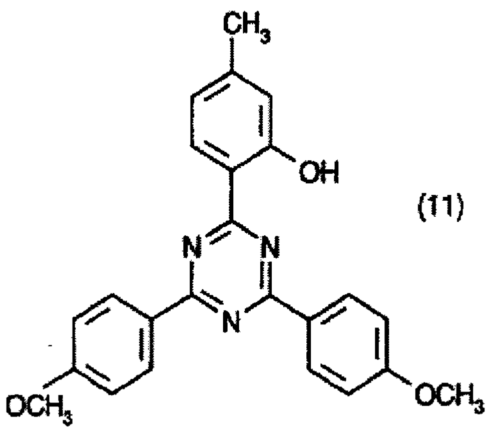

(11)

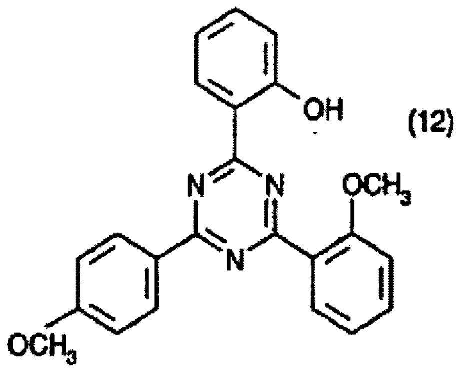

(12)

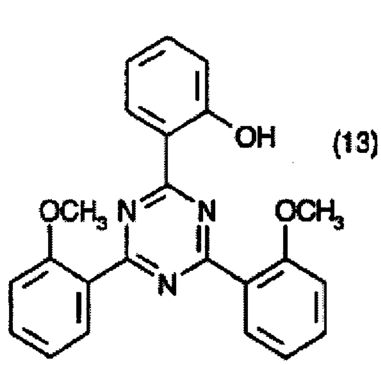

(13)

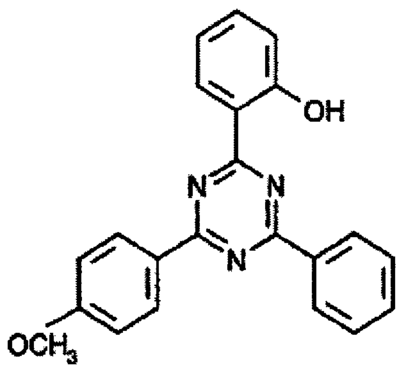

(14)

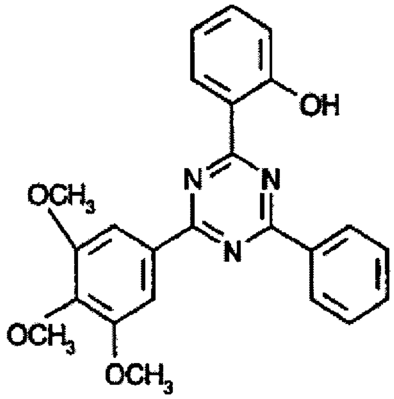

(15)

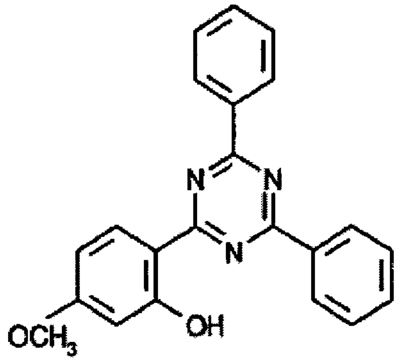

(16)

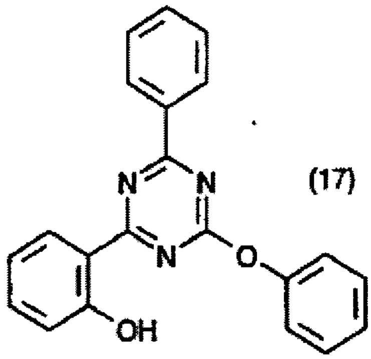

(17)

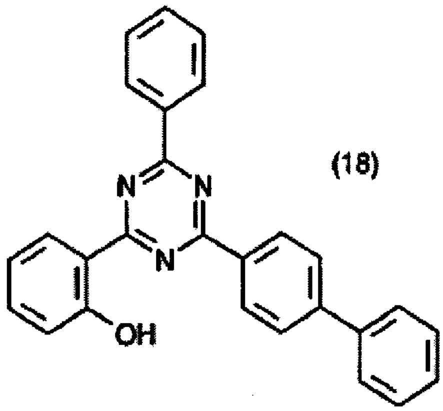

(18)

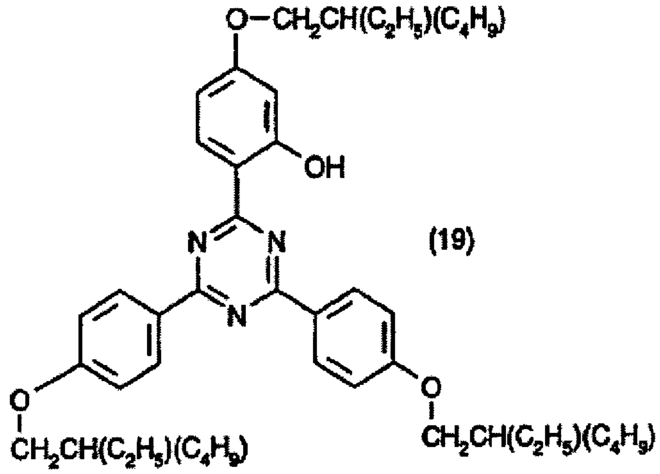

(19)

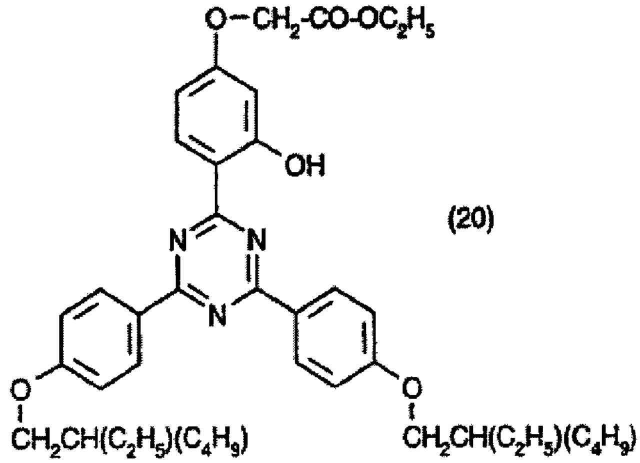

(20)

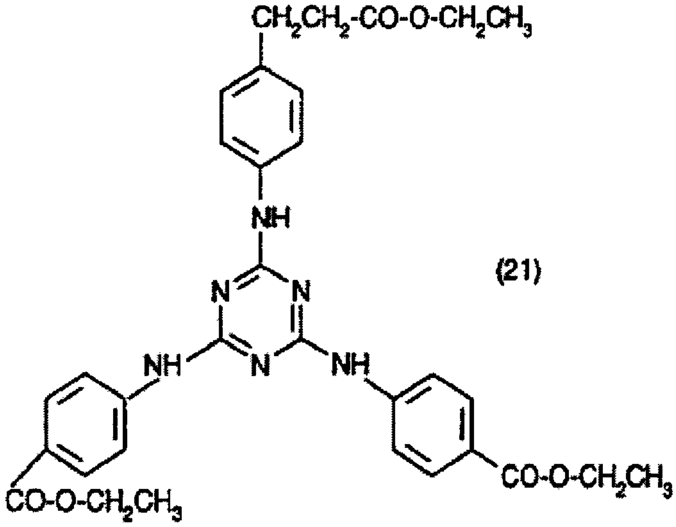

(21)

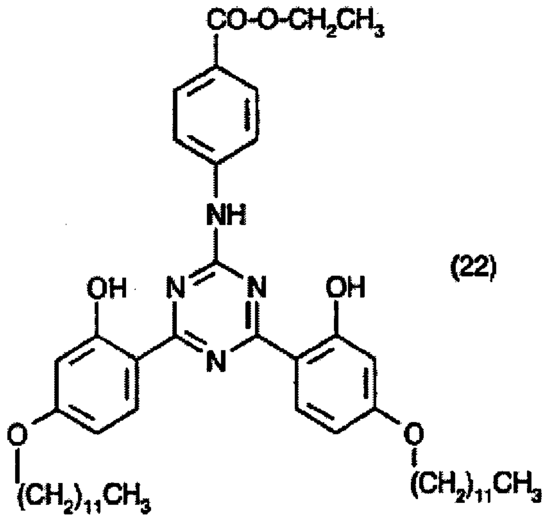

(22)

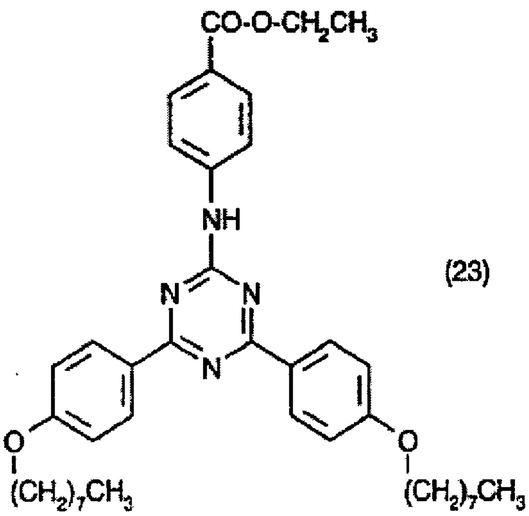

(23)

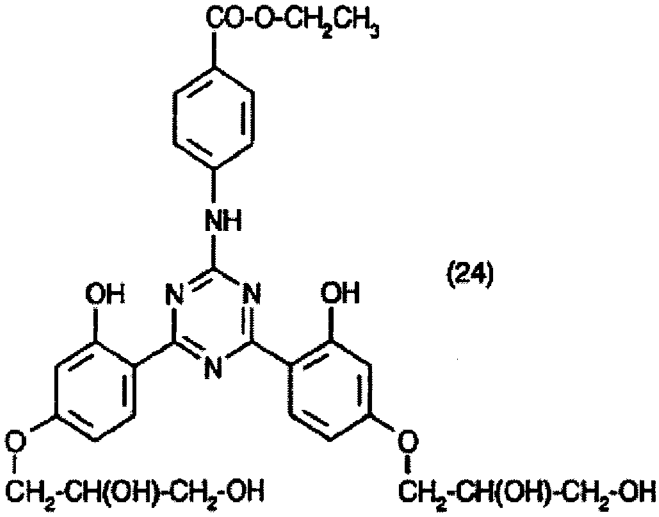

(24)

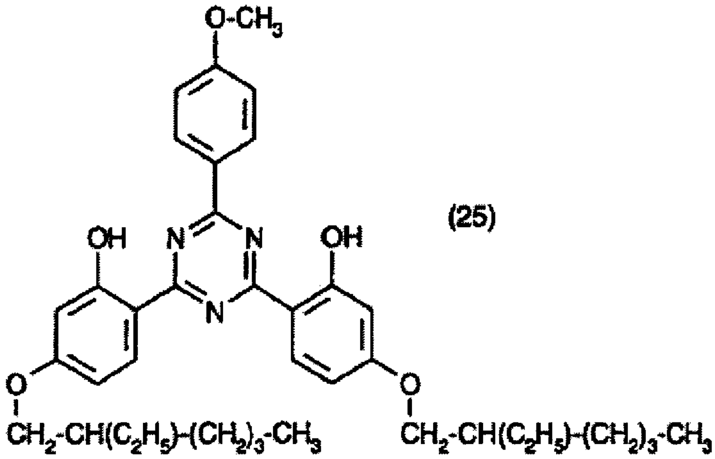

(25)

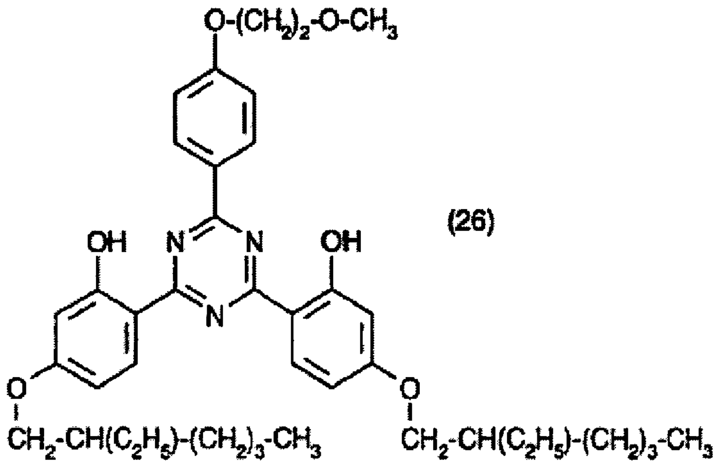

(26)

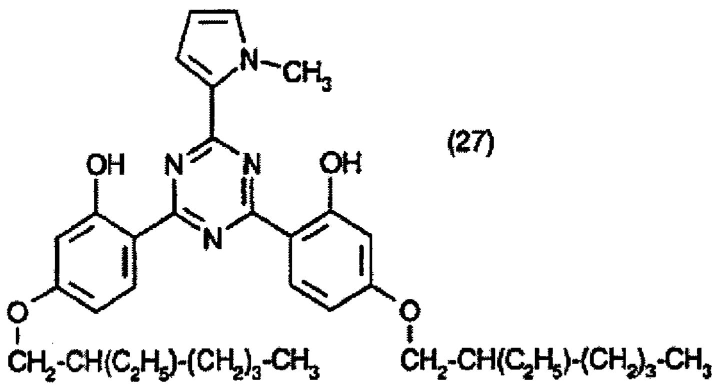

(27)

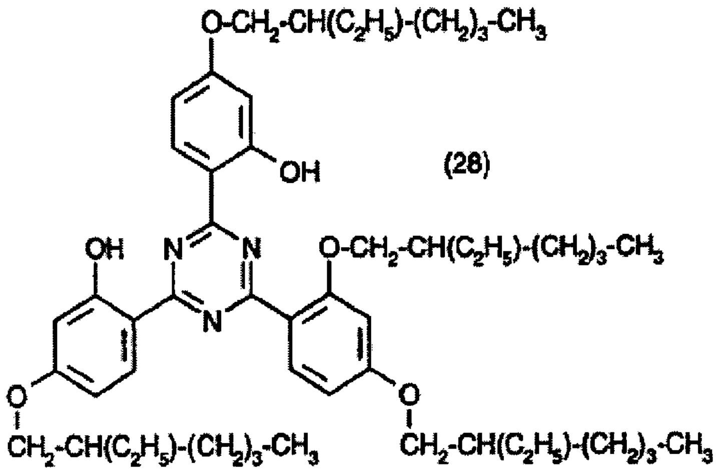

(28)

and

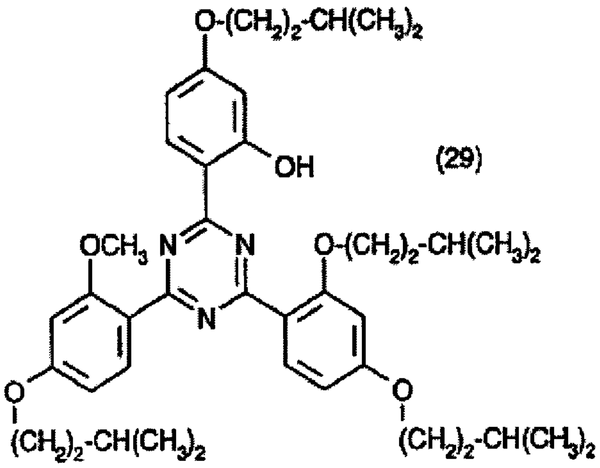

(29)

as well as 2,4,6-tris(diisobutyl-4'-aminobenzalmalonate)-s-triazine and 2,4-bis(diisobutyl-4-aminobenzalmalonate)-6-(4'-aminobenzylidenecamphor)-s-triazine. Bis-ethylhexyloxyphenol methoxyphenyl triazine, marketed under the trademark "Tinosorb S" by Ciba-Geigy is in particular preferable.

Particularly preferred compounds of formula (2) are those having the formula:

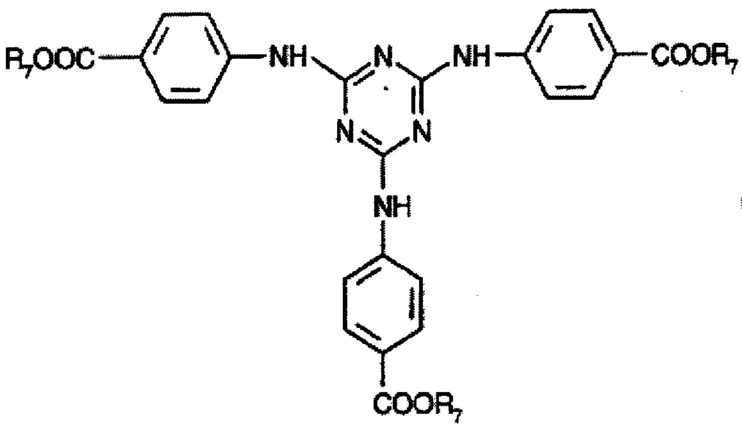

(30)

in which the individual radicals $R_7$ are the same or different and each is hydrogen; an alkali metal; an ammonium group $N(R_8)_4$ in which $R_8$ is hydrogen or an organic radical; $C_1$-$C_{20}$ alkyl; or a polyoxyethylene radical which contains from 1 to 10 ethylene oxide units and the terminal OH group of which may be etherified by a $C_1$-$C_3$ alcohol.

In relation to the compounds of formula (30), when $R_7$ is an alkali metal it is preferably potassium or, especially sodium; when $R_7$ is the group $N(R_8)_4$ in which $R_8$ has its previous meaning, it is preferably a mono-, di- or tri-$C_1$-$C_4$ alkylammonium salt, a mono-, di- or tri-$C_2$-$C_4$ alkanolammonium salt or a $C_1$-$C_{20}$ alkyl ester thereof; when $R_8$ is a $C_1$-$C_{20}$ alkyl group, it is preferably a $C_6$-$C_{12}$ alkyl group, more preferably a $C_8$-$C_9$ alkyl group, especially a 3,5,5-trimethylpentyl group or, most particularly, a 2-ethylhexyl group; and when $R_8$ is a polyoxyethylene group, this preferably contains from 2-6 ethylene oxide units.

A preferred class of solid triazole UV absorbers is that having the formula:

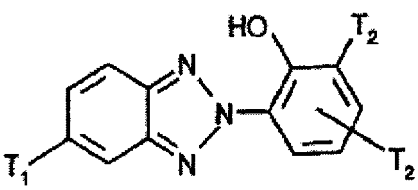

(31)

in which $T_1$ is $C_1$-$C_{18}$ alkyl or, preferably, hydrogen; and $T_2$ is hydrogen, hydroxyl, or $C_1$-$C_{18}$ alkyl, optionally substituted by phenyl, preferably $\alpha,\alpha$-dimethylbenzyl.

A further preferred class of solid triazole UV absorbers is that having the formula:

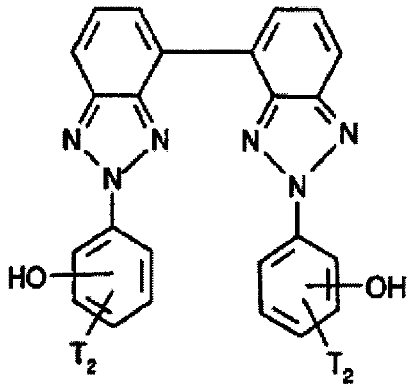

(32)

in which $T_2$ has its previous meaning.

A still further preferred class of solid triazole UV absorbers is that having the formula:

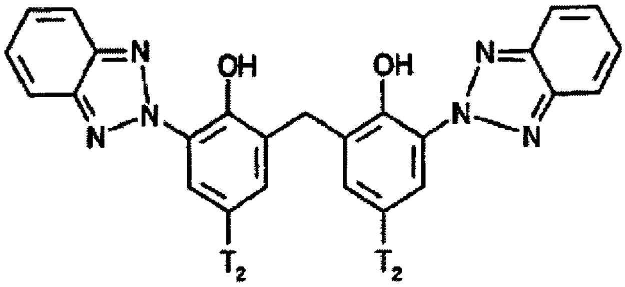

(33)

in which $T_2$ has its previous meaning and is preferably t-butyl.

A preferred class of solid vinyl group-containing amide UV absorbers is that having the formula:

$$R_9\text{-}(Y)_m\text{-CO-C}(R_{10})\text{=C}(R_{11})\text{-N}(R_{12})\ (R_{13}) \qquad (34)$$

in which $R_9$ is $C_1$-$C_{18}$ alkyl, preferably $C_1$-$C_5$ alkyl, or phenyl optionally substituted by one, two or three substituents selected from OH, $C_1$-$C_{18}$ alkyl, $C_1$-$C_{18}$ alkoxy or CO-$OR_6$ in which $R_6$ has its previous meaning; $R_{10}$, $R_{11}$, $R_{12}$ and $R_{13}$ are the same or different and each is $C_1$-$C_{18}$ alkyl, preferably $C_1$-$C_5$ alkyl, or hydrogen; Y is N or O; and m has its previous meaning.

Preferred compounds of formula (34) are 4-octyl-3-penten-2-one, ethyl-3-octylamino-2-butenoate, 3-octylamino-1-phenyl-2-buten-1-one and 3-dodecylamino-1-phenyl-2-buten-1-one.

A preferred class of solid cinnamic acid amide UV absorbers is that having the formula:

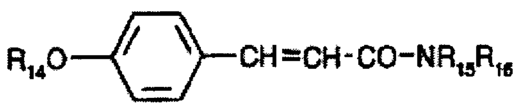

(35)

in which $R_{14}$ is hydroxy or $C_1$-$C_4$ alkoxy, preferably methoxy or ethoxy; $R_{15}$ is hydrogen or $C_1$-$C_4$ alkyl, preferably methyl or ethyl; and $R_{16}$ is -$(CONH)_m$-phenyl in which m has its previous meaning and the phenyl group is optionally substituted by one, two or three substituents selected from OH, $C_1$-$C_{18}$ alkyl, $C_1$-$C_{18}$ alkoxy or CO-$OR_6$ in which $R_6$ has its previous meaning. Preferably $R_{16}$ is phenyl, 4-methoxyphenyl or the phenylaminocarbonyl group.

A preferred class of solid sulfonated benzimidazole UV absorbers is that having the formula:

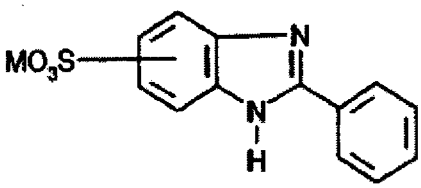

(36)

in which M is hydrogen or an alkali metal, preferably sodium, an alkaline earth metal, such as magnesium or calcium, or zinc.

In the compounds of formula (1) to (35), $C_1$-$C_{18}$ alkyl groups may be methyl, ethyl, n-propyl, isopropyl, n-butyl, isobutyl, tert-butyl, n-amyl, n-hexyl, n-heptyl, n-octyl, iso-octyl, n-nonyl, n-decyl, n-undecyl, n-dodecyl, tetradecyl, hexydecyl or octadecyl; and $C_1$-$C_{18}$ alkoxy groups include methoxy, ethoxy, propoxy, butoxy, n-hexoxy, n-heptoxy, n-octoxy, iso-octoxy, n-nonoxy, n-decoxy, n-undecoxy, n-dodecoxy, tetradecoxy, hexadecoxy or octadecoxy, methoxy and ethoxy being preferred.

$C_1$-$C_{18}$ carboxyalkyl includes carboxymethyl, carboxyethyl, carboxypropyl, carboxyisopropyl, carboxybutyl, carboxyisobutyl, carboxybutyl, carboxyamyl, carboxyhexyl, carboxyheptyl, carboxyoctyl, carboxyisooctyl, carboxynonyl, carboxydecyl, carboxyundecyl, carboxydodecyl, carboxytetradecyl, carboxyhexadecyl and carboxyoctadecyl, carboxymethyl being preferred.

$C_5$-$C_8$ cycloalkyl includes cyclopentyl, cyclohexyl and cyclooctyl.

The compounds of formula (1) to (35) are known. The compounds of formula (30) are described, together with their production, in U.S. Pat. No. 4,617,390.

It is preferable that the organic solid UV filter(s) is a benzotriazole derivative, in particular, a phenylbenzotriazole derivative such as a drometrizole trisiloxane, marketed under the trademark "Silatrizole" by Rhodia Chimie or "Mexoryl XL" by L'Oreal, as represented below.

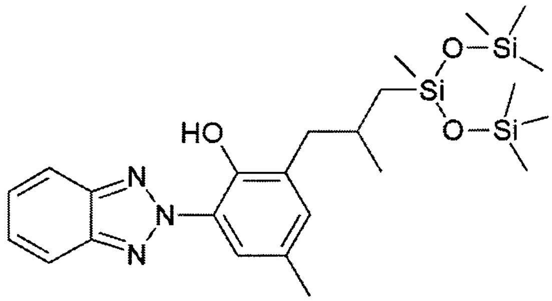

If organic liquid UV filter(s) is/are used as the additional UV filter(s), the additional UV filter(s) may be selected from the group consisting of anthranilic derivatives; dibenzoylmethane derivatives; liquid cinnamic derivatives; salicylic derivatives; camphor derivatives; benzophenone derivatives; β,β-diphenylacrylate derivatives; liquid triazine derivatives; liquid benzotriazole derivatives; benzalmalonate derivatives; benzimidazole derivatives; imidazoline derivatives; bis-benzoazolyl derivatives; p-aminobenzoic acid (PABA) and derivatives thereof; methylenebis(hydroxyphenylbenzotriazole) derivatives; benzoxazole derivatives; screening polymers and screening silicones; dimers derived from α-alkylstyrene; 4,4-diarylbutadienes; octocrylene and derivatives thereof, guaiazulene and derivatives thereof, rutin and derivatives thereof, flavonoids, biflavonoids, oryzanol and derivatives thereof, quinic acid and derivatives thereof, phenols, retinol, cysteine, aromatic amino acids, peptides having an aromatic amino acid residue, and mixtures thereof.

Mention may be made, as examples of the organic liquid UV filter(s), of those denoted below under their INCI names, and mixtures thereof.

Anthranilic derivatives: Menthyl anthranilate, marketed under the trademark "Neo Heliopan MA" by Haarmann and Reimer.

Dibenzoylmethane derivatives: Butyl methoxydibenzoylmethane, marketed in particular under the trademark "Parsol 1789" by Hoffmann-La Roche; and isopropyl dibenzoylmethane.

Liquid cinnamic derivatives: Ethylhexyl methoxycinnamate, marketed in particular under the trademark "Parsol MCX" by Hoffmann-La Roche; isopropyl methoxycinnamate; isopropoxy methoxycinnamate; isoamyl methoxycinnamate, marketed under the trademark "Neo Heliopan E 1000" by Haarmann and Reimer; cinoxate (2-ethoxyethyl-4-methoxy cinnamate); DEA methoxycinnamate; diisopropyl methylcinnamate; and glyceryl ethylhexanoate dimethoxycinnamate.

Salicylic derivatives: Homosalate (homomentyl salicylate), marketed under the trademark "Eusolex HMS" by Rona/EM Industries; ethylhexyl salicylate, marketed under the trademark "Neo Heliopan OS" by Haarmann and Reimer; glycol

salicylate; butyloctyl salicylate; phenyl salicylate; dipropyleneglycol salicylate, marketed under the trademark "Dipsal" by Scher; and TEA salicylate, marketed under the trademark "Neo Heliopan TS" by Haarmann and Reimer.
Camphor derivatives, in particular, benzylidenecamphor derivatives: 3-benzylidene camphor, manufactured under the trademark "Mexoryl SD" by Chimex; 4-methylbenzylidene camphor, marketed under the trademark "Eusolex 6300" by Merck; benzylidene camphor sulfonic acid, manufactured under the trademark "Mexoryl SL" by Chimex; camphor benzalkonium methosulfate, manufactured under the trademark "Mexoryl SO" by Chimex; terephthalylidene dicamphor sulfonic acid, manufactured under the trademark "Mexoryl SX" by Chimex; and polyacrylamidomethyl benzylidene camphor, manufactured under the trademark "Mexoryl SW" by Chimex.
Benzophenone derivatives: Benzophenone-1 (2,4-dihydroxybenzophenone), marketed under the trademark "Uvinul 400" by BASF; benzophenone-2 (Tetrahydroxybenzophenone), marketed under the trademark "Uvinul D50" by BASF; Benzophenone-3 (2-hydroxy-4-methoxybenzophenone) or oxybenzone, marketed under the trademark "Uvinul M40" by BASF; benzophenone-4 (hydroxymethoxy benzophonene sulfonic acid), marketed under the trademark "Uvinul MS40" by BASF; benzophenone-5 (Sodium hydroxymethoxy benzophenone Sulfonate); benzophenone-6 (dihydroxy dimethoxy benzophenone); marketed under the trademark "Helisorb 11" by Norquay; benzophenone-8, marketed under the trademark "Spectra-Sorb UV-24" by American Cyanamid; benzophenone-9 (Disodium dihydroxy dimethoxy benzophenonedisulfonate), marketed under the trademark "Uvinul DS-49" by BASF; benzophenone-12, and n-hexyl 2-(4-diethylamino-2-hydroxybenzoyl)benzoate.
β,β-diphenylacrylate derivatives: Octocrylene, marketed in particular under the trademark "Uvinul N539" by BASF; and Etocrylene, marketed in particular under the trademark "Uvinul N35" by BASF.
Liquid triazine derivatives: diethylhexyl butamido triazone, marketed under the trademark "Uvasorb HEB" by Sigma 3V; 2,4,6-tris(dineopentyl 4'-aminobenzalmalonate)-s-triazine; and the symmetrical triazine screening agents described in U.S. Pat. No. 6,225,467, WO 2004/085412 (see Compounds 6 and 9) or the document "Symmetrical Triazine Derivatives", IP.COM Journal, IP.COM INC, WEST HENRIETTA, NY, US (20 Sep. 2004), in particular the 2,4,6-tris(biphenyl)-1,3,5-triazines (especially 2,4,6-tris(biphenyl-4-yl)-1,3,5-triazine) and 2,4,6-tris(terphenyl)-1,3,5-triazine, which is taken up again in WO 06/035000, WO 06/034982, WO 06/034991, WO 06/035007, WO 2006/034992 and WO 2006/034985.
Liquid benzotriazole derivatives, in particular, phenylbenzotriazole derivatives: 2-(2H-benzotriazole-2-yl)-6-dodecyl-4-methylpheno, branched and linear; and those described in USP 5240975.
Benzalmalonate derivatives: Dineopentyl 4'-methoxybenzalmalonate, and polyorganosiloxane comprising benzalmalonate functional groups, such as polysilicone-15, marketed under the trademark "Parsol SLX" by Hoffmann-LaRoche.
Benzimidazole derivatives, in particular, phenylbenzimidazole derivatives: Phenylbenzimidazole sulfonic acid, marketed in particular under the trademark "Eusolex 232" by Merck, and disodium phenyl dibenzimidazole tetrasulfonate, marketed under the trademark "Neo Heliopan AP" by Haarmann and Reimer.
Imidazoline derivatives: Ethylhexyl dimethoxybenzylidene dioxoimidazoline propionate.
bis-benzoazolyl derivatives: The derivatives as described in EP-669,323 and U.S. Pat. No. 2,463,264.
para-aminobenzoic acid and derivatives thereof: PABA (p-aminobenzoic acid), ethyl PABA, Ethyl dihydroxypropyl PABA, pentyl dimethyl PABA, ethylhexyl dimethyl PABA, marketed in particular under the trademark "Escalol 507" by ISP, glyceryl PABA, and PEG-25 PABA, marketed under the trademark "Uvinul P25" by BASF.
Methylenebis(hydroxyphenylbenzotriazole) derivatives: Methylene bis-benzotriazolyl tetramethylbutylphenol, marketed in the solid form under the trademark "Mixxim BB/100" by Fairmount Chemical or in the micronized form in aqueous dispersion under the trademark "Tinosorb M" by Ciba Specialty Chemicals, and the derivatives as described in U.S. Pat. Nos. 5,237,071, 5,166,355, GB-2,303,549, DE-197,26,184 and EP-893,119.
Benzoxazole derivatives: 2,4-bis[5-1(dimethylpropyl)benzoxazol-2-yl-(4-phenyl)imino]-6-(2-ethylhexyl)imino-1,3,5-triazine, marketed under the trademark of Uvasorb K2A by Sigma 3V.
Screening polymers and screening silicones: The silicones described in WO 93/04665.
Dimers derived from α-alkylstyrene: The dimers described in DE-19855649.
4,4-diarylbutadiene derivatives: 1,1-dicarboxy(2,2'-dimethylpropyl)-4,4-diphenylbutadiene.
Octocrylene and derivatives thereof: Octocrylene.
Quaiazulene and derivatives thereof: Guaiazulene and sodium guaiazulene sulfonate.
Rutin and derivatives thereof: Rutin and glucosylrutin.
Flavonoids: Robustin (isoflavonoid), genistein (flavonoid), tectochrysin (flavonoid), and hispidone (flavonoid).
Biflavonoids: Lanceolatin A, lanceolatin B, and hypnumbiflavonoid A.
Oryzanol and derivatives thereof: r-oryzanol.
Quinic acid and derivatives thereof: Quinic acid.
Phenols: Phenol.
Retinols: Retinol.
Cysteines: L-cysteine.
Peptides having an aromatic amino acid residue: Peptides having tryptophan, tyrosine or phenylalanine.
The preferred organic liquid UV filter(s) is selected from: butyl methoxydibenzoylmethane, ethylhexyl methoxycinnamate,

homosalate, ethylhexyl salicylate, octocrylene, phenylbenzimidazole sulfonic acid, benzophenone-3, benzophenone-4, benzophenone-5, n-hexyl 2-(4-diethylamino-2-hydroxybenzoyl)benzoate, 4-methylbenzylidene camphor, terephthalylidene dicamphor sulfonic acid, disodium phenyl dibenzimidazole tetrasulfonate, ethylhexyl triazone, bis-ethylhexyloxyphenol methoxyphenyl triazine, diethylhexyl butamido triazone, 2,4,6-tris(dineopentyl 4'-aminobenzalmalonate)-s-triazine, 2,4,6-tris(diisobutyl 4'-aminobenzalmalonate)-s-triazine, 2,4,6-tris(biphenyl-4-yl)-1,3,5-triazine, 2,4,6-tris(terphenyl)-1,3,5-triazine, methylene bis-benzotriazolyl tetramethylbutylphenol, polysilicone-15, dineopentyl 4'-methoxybenzalmalonate, 1,1-dicarboxy(2,2'-dimethylpropyl)-4,4-diphenylbutadiene, 2,4-bis[5-1(dimethylpropyl)benzoxazol-2-yl-(4-phenyl)imino]-6-(2-ethylhexyl)imino-1,3,5-triazine, and their mixtures. A more preferable organic liquid UV filter is butyl methoxydibenzoylmethane(Avobenzone).

The additional UV filter(s) may be used in the composite pigment according to the present invention in proportions such that the weight ratio of the small core particle to the additional UV filter(s) is 50:50 to 90:10, preferably 50:50 to 80:20, and more preferably 50:50 to 70:30.

(Method for Preparing Composite Pigment)

The composite pigment according to the present invention can be prepared by subjecting a small core particle of more than 100 nm and less than 1 μm, preferably less than 600 nm, and more preferably less than 400 nm, at least one inorganic solid UV filter and/or at least one coloring pigment, and optionally at least one additional UV filter, to a mechanochemical fusion process.

Mechanochemical fusion process means a process in which mechanical power such as impact force, friction force or shear force is applied to a plurality of subjects to cause fusion between the subjects.

The mechanochemical fusion process may be performed by, for example, an apparatus comprising a rotating chamber and a fixed inner piece with a scraper, such as a mechanofusion system marketed by Hosokawa Micron Corporation in Japan.

It is preferable to use a hybridizer process as the mechanochemical fusion process.

The hybridizer process was developed in the 1980s. The hybridizer process is a class of mechanochemical fusion processes in which strong mechanical power is applied to a plurality of particles to cause a mechanochemical reaction to form a composite particle.

According to the hybridizer process, the mechanical power is imparted by a high speed rotor which can have a diameter from 10 cm to 1 m, and can rotate at a speed of 1,000 rpm to 100,000 rpm. Therefore, the hybridizer process can be defined as a mechanochemical fusion process using such a high speed rotor. The hybridizer process is performed in air or under dry conditions. Thus, due to the high speed rotation of the rotor, high speed air flow may be generated near the rotor. However, some liquid materials may be subjected to the hybridizer process together with solid materials. The term "hybridizer process" has been used as a technical term.

The hybridizer process can be performed by using a hybridization system marketed by, for example, Nara Machinery in Japan, in which at least two types of particles, typically core particles and fine particles, are fed into a hybridizer equipped with a high speed rotor having a plurality of blades in a chamber under dry conditions, and the particles are dispersed in the chamber and mechanical and thermal energy (e.g., compression, friction and shear stress) are imparted to the particles for a relatively short period of time such as 1 to 10 minutes, preferably 1 to 5 minutes. As a result, one type of particles (e.g., fine particles) is embedded or fixed on the other type of particles (e.g., core particle) to form composite particles. It is preferable that the particles have been subjected to electrostatic treatment(s) such as shaking to form an "ordered mixture" in which one type of particles is spread to cover the other type of particles. The hybridizer process can also be performed by using a theta composer marketed by Tokuju Corporation in Japan.

The hybridizer process can also be performed by using a Composi Hybrid or a Mechano Hybrid marketed by Nippon coke.

According to the present invention, a small core particle and inorganic solid UV filter(s) and/or coloring pigment(s) as well as optionally additional material(s) such as additional UV filter(s) if necessary, can be fed into such a hybridizer to form a composite pigment. The hybridizer process can be performed by using a rotor rotating at about 8,000 rpm (100 m/sec) for about 3 minutes.

The small core particle and inorganic solid UV filter(s) and/or coloring pigment(s) can be used in proportions such that the weight ratio of the small core particle to the inorganic solid UV filter(s) and/or coloring pigment(s) is 50:50 to 90:10, preferably 50:50 to 80:20, and more preferably 50:50 to 70:30.

The hybridizer process enables to provide a composite pigment in which a small core particle is at least in part covered by at least one layer comprising at least one inorganic solid UV filter and/or at least one coloring pigment, and optionally additional UV filter(s).

Furthermore, the hybridizer process can provide ordered array (e.g., uniform coverage) of inorganic solid UV filter(s) and/or coloring pigment(s) and optionally additional UV filter(s) on a small core particle and provides strong bonds at the surface of the core particle and a layer comprising the inorganic solid UV filter(s) and/or coloring pigment(s)

and optionally additional UV filter(s).

It should be noted that the hybridizer process is quite different from other processes using, for example, a beads mill and a jet mill. In fact, a beads mill causes pulverization or aggregation of core particles, and a jet mill causes pulverization of core particles and uniform coating of a core particle by fine particles is difficult to be formed.

If necessary, an additional process for further coating the composite pigment with UV filter(s) and/or coloring material(s) may be performed. As a result of this additional process, the composite pigment according to the present invention may be coated with a further layer comprising UV filter(s) and/or coloring material(s), preferably consisting of UV filter(s) and/or coloring material(s).

(Composite Pigment Composition)

The composite pigment according to the present invention may be combined with large particle(s) to form a composite pigment composition.

The composite pigment composition according to the present invention comprises at least one composite pigment according to the present invention, and

at least one large particle with a mean particle size of 2 μm or more, preferably 3 μm or more, more preferably 4 μm or more, and even more preferably 5 μm or more,

wherein the surface of the large particle is optionally at least in part covered with at least one layer comprising at least one inorganic solid UV filter and/or at least one coloring pigment.

The mean particle size of the large particle may be limited to 50 μm or less, preferably 30 μm or less, and more preferably 20 μm or less, and even more preferably 10 μm or less.

The mean particle size or mean particle diameter of the large particle is an arithmetic mean diameter, and can be determined by, for example, calculating the mean or average of the dimensions of one hundred particles chosen on an image obtained with a scanning electron microscope.

The large particle can be in any shape. For example, it is possible to use a large particle in the form of a plate with an aspect ratio of at least 5, preferably more than 10, more preferably more than 20, and more preferably more than 50. The aspect ratio can be determined by the average thickness and the average length according to the formula: aspect ratio = length/thickness.

If a plate-like particle is used for the present invention, it is preferable that the plate-like particle has a length ranging 2 μm or more, preferably 3 μm or more, more preferably 4 μm or more, and even more preferably 5 μm or more, but ranging 50 μm or less, preferably 30 μm or less, and more preferably 20 μm or less, and even more preferably 10 μm or less.

The material of the large particle is not limited. The material can be at least one inorganic material and/or at least one organic material.

The inorganic material and/or organic material may be hollow or porous. The porosity of the material may be characterized by a specific surface area of from 0.05 $m^2/g$ to 1,500 $m^2/g$, more preferably from 0.1 $m^2/g$ to 1,000 $m^2/g$, and more preferably from 0.2 $m^2/g$ to 500 $m^2/g$ according to the BET method. However, it is preferable to use solid inorganic material(s) and/or solid organic material(s), preferably 'not hollow' materials.

The large particle comprises at least one inorganic material and/or at least one organic material, preferably at least one organic material.

Preferably, the inorganic material can be selected from the group consisting of mica, synthetic mica, talc, sericite, boron nitride, glass flakes, calcium carbonate, barium sulfate, titanium oxide, hydroxyapatite, silica, silicate, zinc oxide, magnesium sulfate, magnesium carbonate, magnesium trisilicate, aluminum oxide, aluminum silicate, calcium silicate, calcium phosphate, magnesium oxide, bismuth oxychloride, kaolin, hydrotalcite, mineral clay, synthetic clay, iron oxide, and mixtures thereof. Natural mica, synthetic mica, sericite, kaolin, talc, silica and mixtures thereof are more preferable.

In particular, silica particles such as P-1500 marketed by JGC C&C are preferable as inorganic large particles.

Preferably, the organic material can be selected from the group consisting of poly(meth)acrylates, polyamides, silicones, polyurethanes, polyethylenes, polypropylenes, polystyrenes, polyhydroxyalkanoates, polycaprolactams, poly(butylene) succinates, polysaccharides, polypeptides, polyvinyl alcohols, polyvinyl resins, fluoropolymers, waxes, amidosulfonic acid polyvalent metal salts, acylated amino acids, and mixtures thereof. As the fluoropolymers, for example, PTFE may be used. As the amidosulfonic acid polyvalent metal salts, for example, N-lauroyltaurine calcium may be used. As the acylated amino acids, lauroyllysine may be used. Polyamides such as Nylon®, polyhydroxyalkanoates such as polylactic acids, poly(meth)acrylates such as polymethylmethacrylates, silicones, fluoropolymers, and mixtures thereof are more preferable.

In particular, polyamide particles such as SP-500 marketed by Toray and Orgasol marketed by Arkema, and PTFE particles such as Ceridust 9205F marketed by Clariant are preferable as organic large particles.

In a preferred embodiment, the large particle is selected from polyamide particles.

The large particle may or may not be coated beforehand. In a particular embodiment, the large particle is coated. The material of a coating of the large particle is not limited, but an organic material such as an amino acid, an N-acylamino acid, an amido, a silicone, a modified silicone and a polyolefin, is preferable. As the organic material, mention may be made of lauroyl lysine, acryl-modified silicone and polyethylene.

In particular, silica particles coated with polyethylene such as ACEMATT OK412 marketed by Degussa are preferable as coated (inorganic) large particles.

In the composite pigment composition, the weight ratio of the small core particle(s) to the large particle(s) may be 10:90 to 90:10, preferably 20:80 to 80:20, and more preferably 30:70 to 70:30.

The weight ratio of the small particle(s)/the large particle(s)/the inorganic solid UV filter(s) may be 9:81:10 to 27:3:70, preferably 8:72:20 to 45:5:50, and more preferably 7:63:30 to 63:7:30.

In a particular embodiment, the weight ratio of the small particle(s)/the large particle(s)/the inorganic solid UV filter(s) may be 20:50:30 to 50:20:30, preferably 35:15:50 to 15:35:50.

In a preferred embodiment, the weight ratio of the small particle(s)/the large particle(s)/the inorganic solid UV filter(s) may be 35:35:30.

(Method for Preparing Composite Pigment Composition)

The composite pigment composition according to the present invention can be prepared by subjecting at least one small core particle of more than 100 nm and less than 1 μm, preferably less than 600 nm, and more preferably less than 400 nm, at least one inorganic solid UV filter and/or at least one coloring pigment, at least one large particle with a mean particle size of 2 μm or more, preferably 3 μm or more, more preferably 4 μm or more, and even more preferably 5 μm or more, and optionally at least one additional UV filter, to a mechanochemical fusion process as explained above.

The small core particle, the large particle, the inorganic solid UV filter, the coloring pigment, and the additional UV filter are as explained above.

In the mechanochemical fusion process, preferably a hybridizer process using a hybridization system marketed by, for example, Nara Machinery in Japan, the small core particle(s) and the large particle(s) can be used in proportions such that the weight ratio of the small core particle to the large particle(s) is 10:90 to 90:10, preferably 20:80 to 80:20, and more preferably 30:70 to 70:30.

The weight ratio of the small particle(s)/the large particle(s)/the inorganic solid UV filter(s) may be 9:81:10 to 27:3:70, preferably 8:72:20 to 45:5:50, and more preferably 7:63:30 to 63:7:30.

In a particular embodiment, the weight ratio of the small particle(s)/the large particle(s)/the inorganic solid UV filter(s) may be 20:50:30 to 50:20:30, preferably 35:15:50 to 15:35:50.

In a preferred embodiment, the weight ratio of the small particle(s)/the large particle(s)/the inorganic solid UV filter(s) may be 35:35:30.

When the large particle(s) are used in combination with the small core particle(s), the inorganic solid UV filter and/or the coloring pigment, and optionally the additional UV filter, can be effectively bound on the surface of the small core particle(s) due to the anchor effects by the collision of the large particle(s) to the small core particle(s). Therefore, the UV filtering effects and/or coloring effects can be further enhanced.

Accordingly, it is preferable that the small core particle(s) be combined with large particle(s) to prepare a composite pigment composition according to the present invention.

(Cosmetic Composition)

The composite pigment or composite pigment composition, as described above, according to the present invention can be present in the cosmetic composition according to the present invention in an amount ranging from 0.01% to 99% by weight, preferably 0.1% to 50% by weight, and more preferably 1% to 30% by weight, relative to the total weight of the composition.

Preferably, the composite pigment or the composite pigment composition according to the present invention can be used in cosmetic compositions to be applied to keratin substances such as skin, hair, and nails, providing superior UV shielding effects, and/or coloring effects, because the composite pigment or the composite pigment composition can exhibit good UV filtering effects possibly with a transparent or clear appearance and/or good coloring effects such as more transparent or clearer and more bright coloring, without the risk of affecting keratin substances.

Since the composite pigment or the composite pigment composition according to the present invention can reduce free particles which have a high friction coefficient such that they do not easily spread on the skin and provide an unpleasant feeling on use, the cosmetic composition according to the present invention has reduced friction, and therefore, can provide the effect of a better feeling on use.

The cosmetic composition according to the present invention may further comprise at least one filler and/or at least one oil.

As used herein, the term "filler" should be understood as meaning colorless natural or synthetic particles of any shape which are insoluble in the medium of the composition, whatever the temperature at which the composition is manufactured. Thus, the filler is different from the coloring pigment as described above.

The fillers may be inorganic or organic and of any shape(for instance, platelet, spherical, and oblong shapes) and with any crystallographic form (for example, sheet, cubic, hexagonal, orthorhombic, and the like). Examples of suitable additional fillers include, but are not limited to, talc; mica; silica; kaolin; powders of polyamide such as Nylon®: poly-β-3-alanine powders; polyethylene powders; polyurethane powders, such as the powder formed of hexamethylene diisocyanate and trimethylol hexyllactone copolymer sold under the name Plastic Powder D-400 by Toshiki; the powders formed of tetrafluoroethylene polymers (Teflon®); lauroyllysine; starch; boron nitride; polymeric hollow microspheres, such as microspheres of poly(vinylidene chloride)/acrylonitrile, for example Expancel® (Nobel Industrie), and microspheres of acrylic acid copolymers; silicone resin powders, for example, silsesquioxane powders (for instance, silicone resin powders disclosed in European Patent No. 0 293 795 and Tospearls® from Toshiba); poly(methyl methacrylate) particles; precipitated calcium carbonate; magnesium carbonate; basic magnesium carbonate; hydroxyapatite; hollow silica microspheres; glass microcapsules; ceramic microcapsules; metal soaps derived from organic carboxylic acids comprising from 8 to 22 carbon atoms, for example, from 12 to 18 carbon atoms, such as zinc stearate, magnesium stearate, lithium stearate, zinc laurate, and magnesium myristate; barium sulphate; and mixtures thereof.

The filler may be present in the composition in an amount ranging from 0.1% to 80% by weight, with respect to the total weight of the composition, for example, from 1% to 25% by weight, or from 3% to 15% by weight.

The term "oil" is understood to mean a fatty substance which is liquid at ambient temperature (25°C).

Use may be made, as oils which can be used in the composition of the invention, for example, of hydrocarbon oils of animal origin, such as perhydrosqualene (or squalane); hydrocarbon oils of vegetable origin, such as triglycerides of caprylic/capric acids, for example those marketed by Stearineries Dubois or those marketed under the trademarks Miglyol 810, 812 and 818 by Dynamit Nobel, or oils of vegetable origin, for example sunflower, maize, soybean, cucumber, grape seed, sesame, hazelnut, apricot, macadamia, arara, coriander, castor, avocado or jojoba oil or shea butter oil; synthetic oils; silicone oils, such as volatile or non-volatile polymethylsiloxanes (PDMSs) comprising a linear or cyclic silicone chain which are liquid or paste at ambient temperature; fluorinated oils, such as those which are partially hydrocarbon and/or silicone, for example those described in JP-A-2-295912; ethers, such as dicaprylyl ether (CTFA name); and esters, such as benzoate $C_{12}$-$C_{15}$ fatty alcohols (Finsolv TN from Finetex); arylalkyl benzoate derivatives, such as 2-phenylethyl benzoate (X-Tend 226 from ISP); amidated oils, such as isopropyl N-lauroylsarcosinate (Eldew SL-205 from Ajinomoto), and their mixtures.

The oily phase can also comprise one or more fatty substances selected, for example, from fatty alcohols (cetyl alcohol, stearyl alcohol, cetearyl alcohol), fatty acids (stearic acid) or waxes (paraffin wax, polyethylene waxes, carnauba wax, beeswax). The oily phase can comprise lipophilic gelling agents, surfactants or also organic or inorganic particles.

The oily phase can preferably represent from 1 to 70% of oil by weight, with respect to the total weight of the composition.

The composition according to the present invention may further comprise at least one additional conventional cosmetic ingredient which may be chosen, for example, from hydrophilic or lipophilic gelling and/or thickening agents, surfactants, antioxidants, fragrances, preservatives, neutralizing agents, sunscreens, vitamins, moisturizing agents, self-tanning compounds, antiwrinkle active agents, emollients, hydrophilic or lipophilic active agents, agents for combating pollution and/or free radicals, sequestering agents, film-forming agents, dermo-decontracting active agents, soothing agents, agents which stimulate the synthesis of dermal or epidermal macromolecules and/or which prevent their decomposition, antiglycation agents, agents which combat irritation, desquamating agents, depigmenting agents, antipigmenting agents, propigmenting agents, NO-synthase inhibitors, agents which stimulate the proliferation of fibroblasts and/or keratinocytes and/or the differentiation of keratinocytes, agents which act on microcirculation, agents which act on energy metabolism of the cells, healing agents, and mixtures thereof.

It will be possible especially to choose the additional active agents from moisturizers, desquamating agents, agents for improving the barrier function, depigmenting agents, antioxidants, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing the activity of the sebaceous glands, agents for stimulating the energy metabolism of cells, tensioning agents, lipid restructuring agents, slimming agents, agents for promoting the cutaneous microcirculation, calmatives and/or anti-irritants, sebo-regulating or anti-seborrhoeic agents, astringents, cicatrizing agents, anti-inflammatory agents, anti-acne agents and agents which promote natural colouring of the skin.

A person skilled in the art will select the said active agent or agents as a function of the desired effect on the skin, hair, eyelashes, eyebrows or nails.

For caring for and/or making up skin which has aged, the person skilled in the art will preferably select at least one active agent chosen from moisturizers, desquamating agents, agents for improving the barrier function, depigmenting

agents, antioxidants, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing the activity of the sebaceous glands, agents for stimulating the energy metabolism of cells, lipid restructuring agents, agents promoting the cutaneous microcirculation for the area around the eyes and agents which promote the natural colouring of the skin.

For caring for and/or making up greasy skin, the person skilled in the art will preferably select at least one active agent chosen from desquamating agents, sebo-regulating or antiseborrhoeic agents and astringents.
According to a preferred embodiment, the cosmetic and/or dermatological active is a Depigmenting agent.

As depigmenting agents that can be used in accordance with the present invention, mention may in particular be made of vitamin C and derivatives thereof, and in particular vitamin CG, vitamin CP and 3-0 ethyl vitamin C; arbutin and derivatives thereof, such as those described in applications EP895779 and EP524109, for instance alpha- and beta-arbutin; hydroquinone; aminophenol derivatives such as those described in applications WO 99/10318 and WO 99/32077, and in particular N-cholesteryl oxycarbonyl-para-aminophenol and N-ethyloxycarbonyl-para-aminophenol; iminophenol derivatives such as those described in application WO 99/22707; L-2-oxothiazolidine-4-carboxylic acid or procysteine and also salts or esters thereof; ferulic acid; lucinol and derivatives thereof; kojic acid; resorcinol and esters thereof; tranexamic acid and esters thereof; gentisic acid, homogentisate, or methyl gentisate or homogentisate; dioic acid; calcium D-pantethein sulphonate; lipoic acid; ellagic acid; vitamin B3; linoleic acid and derivatives thereof; ceramides and homologues thereof; derivatives of plants, for instance camomile, bearberry, the aloe family (vera, ferox, bardensis), mulberry or skullcap; a kiwi fruit (*Actinidia chinensis)* juice sold by Gattefosse; an extract of *Paeonia suffructicosa* root, such as the product sold by the company Ichimaru Pharcos under the name Botanpi Liquid B®, an extract of brown sugar (*Saccharum officinarum*)*,* such as the extract of molasses sold by the company Taiyo Kagaku under the name Molasses Liquid, without this list being exhaustive. Mention may also be made of biphenyl compounds such as magnolol, honokiol, magnolignan, etc,

We can also mention hydroxylated diphenylmethane derivatives as those described in application WO 2004/105736 and particularly the compound of structure:

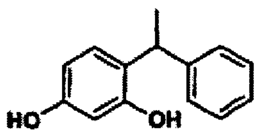

known as 4-(1-phenylethyl)-1,3-benzenediol or 4-(1-phenylethyl)-1,3-dihydroxybenzene or otherwise known as phenylethyl resorcinol or phenylethylbenzenediol or styryl resorcinol. This compound has a CAS number 85-27-8. Such a compound is sold under the name Symwhite 377® by the company Symrise.

Mention may be made especially of:

- a self-tanning agent, i.e. an agent which, when applied to the skin, especially to the face, can produce a tan effect that is more or less similar in appearance to that which may result from prolonged exposure to the sun (natural tan) or under a UV lamp;
- an additional colouring agent, i.e. any compound that has a particular affinity for the skin, which allows it to give the skin a lasting, non-covering coloration (i.e. that does not have a tendency to opacify the skin) and that is not removed either with water or using a solvent, and that withstands both rubbing and washing with a solution containing surfactants. Such a lasting coloration is thus distinguished from the superficial and transient coloration provided, for example, by a makeup pigment; and mixtures thereof.

The self-tanning agents may be chosen from

(i) the compounds interfering with the melanogenesis biological pathway to potentiate it such as for example tyrosinase substrate, MC1R agonists ;

(ii) the monocarbonyl or polycarbonyl compounds, for instance isatin, alloxan, ninhydrin, glyceraldehyde, mesotartaric aldehyde, glutaraldehyde, erythrulose, pyrazoline-4,5-dione derivatives as described in patent application FR 2 466 492 and WO 97/35842, dihydroxyacetone (DHA) and 4,4-dihydroxypyrazolin-5-one derivatives as described in patent application EP 903 342. DHA will preferably be used.

The DHA may be used in free and/or encapsulated form, for example in lipid vesicles such as liposomes,

especially described in patent application WO 97/25970.

In general, the self-tanning agent is present in an amount ranging from 0.01% to 20% by weight and preferably in an amount of between 0.1% and 10% of the total weight of the composition.

Other dyes that allow modification of the colour produced by the self-tanning agent may also be used.

These dyes may be chosen from synthetic or natural direct dyes.

These dyes may be chosen, for example, from red or orange dyes of the fluoran type such as those described in patent application FR 2 840 806. Mention may be made, for example, of the following dyes:

- tetrabromofluorescein or eosin known under the CTFA name: CI 45380 or Red 21;
- phloxin B known under the CTFA name: CI 45410 or Red 27;
- diiodofluorescein known under the CTFA name: CI 45425 or Orange 10;
- dibromofluorescein known under the CTFA name: CI 45370 or Orange 5;
- the sodium salt of tetrabromofluorescein known under the CTFA name: CI 45380 (Na salt) or Red 22;
- the sodium salt of phloxin B known under the CTFA name: CI 45410 (Na salt) or Red 28;
- the sodium salt of diiodofluorescein known under the CTFA name: CI 45425 (Na salt) or Orange 11;
- erythrosine known under the CTFA name: CI 45430 or Acid Red 51;
- phloxin known under the CTFA name: CI 45405 or Acid Red 98.

These dyes may also be chosen from anthraquinones, caramel, carmine, carbon black, azulene blues, methoxalene, trioxalene, guajazulene, chamuzulene, Bengal rose, cosin 10B, cyanosin and daphinin.

These dyes may also be chosen from indole derivatives, for instance the monohydroxyindoles as described in patent FR 2 651 126 (i.e.: 4-, 5-, 6- or 7-hydroxyindole) or the dihydroxyindoles as described in patent EP-B-0 425 324 (i.e.: 5,6-dihydroxyindole, 2-methyl-5,6-dihydroxyindole, 3-methyl-5,6-dihydroxyindole or 2,3-dimethyl-5,6-dihydroxyindole).

The cosmetic and/or dermatological active agents will be present in one of the compositions according to the invention in a content ranging from 0.001% to 20% by weight relative to the total weight of the composition, preferably from 0.01% to 10%, more preferably still from 0.5 to 5% and more preferably from 0.1 to 1% by weight relative to the total weight of the composition.

The composition according to the present invention may be in various forms, for example, suspensions, dispersions, solutions, gels, emulsions, such as oil-in-water (O/W), water-in-oil (W/O), and multiple (e.g., W/O/W, polyol/O/W, and O/W/O) emulsions, creams, foams, sticks, dispersions of vesicles, for instance, of ionic and/or nonionic lipids, two-phase and multi-phase lotions, sprays, powders, and pastes. The composition may be anhydrous, for example, it can be an anhydrous paste or stick. The composition may also be a leave-in composition.

According to a particular embodiment of the invention the compositions according to the invention are in the form of an oil-in-water or water-in-oil emulsion.

The emulsification processes that may be used are of the paddle or impeller, rotor-stator or HHP type.

It is also possible, via HHP (between 50 and 800 bar), to obtain stable dispersions with droplet sizes that may be as low as 100 nm.

The emulsions generally contain at least one emulsifier chosen from amphoteric, anionic, cationic and nonionic emulsifiers, used alone or as a mixture. The emulsifiers are appropriately chosen according to the emulsion to be obtained (W/O or O/W).

As emulsifying surfactants that may be used for the preparation of the W/O emulsions, examples that may be mentioned include sorbitan, glycerol or sugar alkyl esters or ethers; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R by the company Evonik, and the mixture of cetyldimethicone copolyol, of polyglyceryl isostearate (4 mol) and of hexyl laurate, sold under the name Abil WE 09 by the company Evonik. One or more co-emulsifiers may also be added thereto, which may be chosen advantageously from the group comprising polyol alkyl esters.

Polyol alkyl esters that may especially be mentioned include polyethylene glycol esters, for instance PEG-30 dipolyhydroxystearate, such as the product sold under the name Arlacel P135 by the company Croda.

Glycerol and/or sorbitan esters that may especially be mentioned include, for example, polyglyceryl isostearate, such as the product sold under the name Isolan GI 34 by the company Evonik, sorbitan isostearate, such as the product sold under the name Arlacel 987 by the company Croda, sorbitan glyceryl isostearate, such as the product sold under the name Arlacel 986 by the company Croda, and mixtures thereof.

Emulsifying polyoxyalkylenated silicone elastomers may especially be also mentioned as those disclosed in the documents US-A-5,236,986, US-A-5,412,004,
US-A-5,837,793, US-A-5,811,487.Those silicone elastomers are preferably formulated under the form of a gel in a

hydrocarbonated and/or a silicone oil. In those gels, the polyoxyalkylenated silicone elastomer is often under the form of spherical particles.

As example of polyoxyethylenated silicone elastomer, may be mentioned those sold by the company Shin Etsu, with the denominations :

- KSG-21 (at 27 % in active material) INCI name: Dimethicone /PEG-10 Dimethicone vinyl dimethicone crosspolymer),
- KSG-20 (at 95% % in active material) INCI name:. PEG-10 Dimethicone Crosspolymer),
- KSG-30, (at 100 % % in active material) INCI name : Lauryl PEG-15 Dimethicone vinyl dimethicone crosspolymer),
- KSG-31 (at 25 % % in active material) INCI name : Lauryl PEG-15 Dimethicone vinyl dimethicone crosspolymer),
- KSG-32 or KSG-42 or KSG-320 ou KSG-30 (at 25 % % in active material) INCI name : Lauryl PEG-15 Dimethicone vinyl dimethicone crosspolymer),
- KSG-33 (at 20 % in active material),
- KSG-210 (at 25 % % in active material) INCI name : Dimethicone /PEG-10/15 crosspolymer),
- KSG-310 : lauryl modified polydimethylsiloxane polyoxyethylenated in mineral oil,
- KSG-330,
- KSG-340,
- X-226146 (at 32 % % in active material) INCI name :

  Dimethicone /PEG-10 Dimethicone vinyl dimethicone crosspolymer), or those sold by the company Dow Corning under the commercial names : :

  - DC9010 (at 9% % in active material) INCI name : PEG-12 dimethicone crosspolymer)
  - DC9011 at 11% % in active material.

Those products are generally in the form of oily gel containing the particles of silicone elastomer.

Preferably, KSG-210 is used (INCI name : Dimethicone /PEG-10/15 crosspolymer) which is at 25% in active material of silicone elastomer in a silicone oil.

Amongst water/oil emulsifiers, may be mentioned also the polyglycerolated silicone elastomers as those disclosed in the document WO-A-2004/024798.
As example of polyglycerolated silicone elastomers, may be mentioned those sold par the company Shin Etsu, with the denominations :

- KSG-710, (at 25% in active material. INCI name : Dimethicone / Polyglycerin-3 Crosspolymer),
- KSG-810,
- KSG-820,
- KSG-830,
- KSG-840,

For the O/W emulsions, examples of emulsifiers that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters, for instance the mixture PEG-100 stearate/glyceryl stearate sold, for example, by the company Croda under the name Arlacel 165; oxyalkylenated (oxyethylenated and/or oxypropylenated) fatty alkyl ethers; sugar esters, for instance sucrose stearate; fatty alkyl ethers of sugars, especially alkyl polyglucosides (APG) such as decylglucoside and laurylglucoside sold, for example, by the company Cognis under the respective names Plantaren 2000 and Plantaren 1200, cetostearyl glucoside optionally as a mixture with cetostearyl alcohol, sold, for example, under the name Montanov 68 by the company SEPPIC, under the name Tegocare CG90 by the company Evonik and under the name Emulgade KE3302 by the company Cognis, and also arachidyl glucoside, for example in the form of a mixture of arachidyl alcohol, behenyl alcohol and arachidyl glucoside, sold under the name Montanov 202 by the company SEPPIC. According to a specific embodiment of the invention, the mixture of the alkyl polyglucoside as defined above with the corresponding fatty alcohol can be in the form of a self-emulsifying composition, for example as disclosed in the document WO-A-92/06778 ; the hydrophobically modified inulines as Inuline Lauryl Carbamate as the product sold under the denomination INUTEC SP1 by the Company Beneo-ORAFTI.

According to a specific embodiment of the invention, the composition may also contain at least an emulsifier chosen among dimers surfactants named « gemini surfactants » and comprising two surfactant moieties identical or different, and constituted by an hydrophilic head group and a lipophilic linked to each others through the head groups, thanks to a spacer. Such surfactants are described in the patents DE19943681, DE19943668, DE 42 27 391 et DE 196 08 117 ; JP-A-11-60437 ; JP-A-8-311003 ; EP 0 697 244 ; EPO 697 245 ; EP0708 079 ; DE19622612 and JP-A

10-17593 ; WO 03024412, US5863 886 ; WO96/25388 ; WO96/14926 ; WO 96/16930, WO 96/25384WO9740124; WO9731890 ; ; DE19750246; DE 19750245 ; DE 19631225 ; DE 19647060. In order to have a more detailed description of the chemical structures and physicochemical properties, one can refer to the following publications : Milton J. Rosen, Gemini Surfactants, Properties of surfactant molecules with two hydrophilic groups and two hydrophobic groups, Cosmetics & Toiletries magazine, vol. 113, December 1998, pages 49 - 55, Milton J. Rosen, Recent Developments in Gemini Surfactants, Allured's Cosmetics & Toiletries magazine, July 2001, vol 116, n° 7, pages 67 - 70.

Among the dimers surfactant described above, the preferred compounds of the invention are anionic surfactants characterized by the following formula (I)

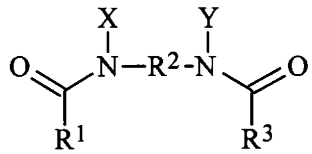

(I)

where

$R^1$ and $R^3$ represent a $C_8$-$C_{16}$ linear alkyl group,
$R^2$ represents a $C_2$-$C_8$ alkylene group,
X and Y represent an $(C_2H_4O)_x$-RF with x = 10 - 15,
and RF = $-SO_3M$ group where M represent an alkaline atom.

A preferred gemini surfactant is an anionic compound Sodium Dicocoyl ethylene diamine PEG-15 Sulfate (nom INCI) with formula :

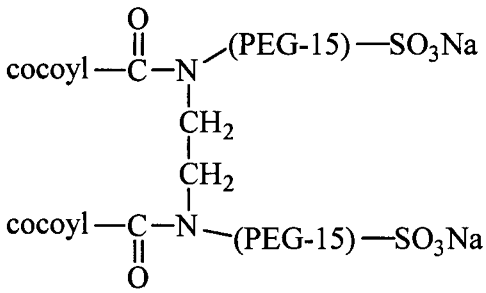

One can use for example this gemini surfactant in the commercialized mixtures sold by Sasol company under the name CERALUTION® :

- Ceralution® H : Behenyl Alcohol, Glyceryl Stearate, Glyceryl Stearate Citrate et Sodium Dicocoyl ethylenediamine PEG-15 Sulfate.
- Ceralution® F : Sodium Lauroyl Lactylate et Sodium Dicocoyl ethylenediamine PEG-15 Sulfate.
- Ceralution® C : Aqua, Capric/Caprylic triglyceride, Glycerine, Ceteareth-25, Sodium Dicocoyl ethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben (INCI denominations).

The preferred gemini surfactant is the mixture of Behenyl Alcohol, Glyceryl Stearate, Glyceryl Stearate Citrate and Sodium Dicocoyl ethylenediamine PEG-15 Sulfate (Ceralution® H).

Among other emulsifiers, may be used isophthalic acid polymers or sulfo isophthalic acid polymers, and specifically copolymers of phthalate / sulfo isophthalate / glycol as for example Diethylene Glycol / Phthalate / Isophthalate / 1,4-cyclohexanedimethanol copolymer (INCI name: Polyester-5sol under the name « Eastman AQ polymer » (AQ35S, AQ38S, AQ55S, AQ48 Ultra) by the company Eastman Chemical.

Among other emulsifiers, amphiphilic copolymers of 2-acrylamido 2-methylpropane sulfonic acid as those described in the patent EP1069142, can be used. The preferred amphiphilic AMPS copolymers are AMMONIUM ACRYLOYLDIMETHYLTAURATE / STEARETH-25 METHACRYLATE CROSSPOLYMER sold under the name Aristoflex HMS by the Company Clariant, AMMONIUM ACRYLOYLDIMETHYLTAURATE / STEARETH-8 METHACRYLATE COPOLYMER sold under the name Aristoflex SNC by the company Clariant.

When it is an emulsion, the aqueous phase of this emulsion may comprise a nonionic vesicular dispersion prepared according to known processes (Bangham, Standish and Watkins, J. Mol. Biol. 13, 238 (1965), FR 2 315 991 and FR 2 416 008).

The compositions of the invention may also contain at least one crosslinked non-emulsifying elastomer organo polysiloxane.

The term « non-emulsifying elastomer organo polysiloxane» means an emulsifying elastomer organo polysiloxane which does not contain any hydrophilic chain as polyoxyalkylenated or polyglycerolated units.

Preferably, the non-emulsifying elastomer organo polysiloxane is obtained by addition reaction (a) of diorgano polysiloxane containing at least two hydrogen atoms each linked to a silicium atom and (b) of diorgano polysiloxane having at least two insaturated ethylenic groups linked to the silicium atom., in particular in presence (c) of a platinium catalyst as disclosed in the application EP-A-295886.

According to particular form of the invention, the non-emulsifying elastomer organopolysiloxane is under the form of powder.

As examples of non-emulsifying elastomer organopolysiloxanes under the form of powder, may be used those having the INCI name : DIMETHICONE/VINYL DIMETHICONE CROSSPOLYMER as the commercial products sold under the names "DOW CORNING 9505 COSMETIC POWDER", "DOW CORNING 9506 COSMETIC POWDER " by the company DOW CORNING.

According a preferred embodiment of the invention, the non-emulsifying elastomer organopolysiloxane is mixed with at least one volatile or non-volatile hydrocarbonated and/or volatile or non-volatile silicone oil for forming a gel.

As examples of mixtures of oil/ non-emulsifying elastomer organopolysiloxane, may be used those having the following INCI names:

DIMETHICONE AND DIMETHICONE/VINYL DIMETHICONECROSSPOLYMER as the commercial products sold under the name « KSG6 », « KSG16 » by the company SHIN ETSU ,

- CYCLOPENTASILOXANE AND DIMETHICONE/VINYL DIMETHICONE CROSSPOLYMER as the commercial products sold under the name "KSG-15", "KSG 24" by the company SHIN ETSU ; « Dow Corning 9040 Silicone Elastomer Blend » by the company DOW CORNING ;
- DIMETHICONE AND DIMETHICONE CROSSPOLYMER as the commercial products sold under the name « Dow Corning 9041 Silicone Elastomer Blend » by the company DOW CORNING ;
- MINERAL OIL AND Vinyl Dimethicone/Lauryl Dimethicone Crosspolymer as "KSG 41" by the company SHIN ETSU
- ISODODECANE AND Vinyl Dimethicone/Lauryl Dimethicone Crosspolymer as "KSG 42" sold by the company SHIN ETSU
- TRIETHYLHEXANOIN AND VINYL DIMETHICONE/LAURYL DIMETHICONE CROSSPOLYMER as « KSG 43 » sold by the company SHIN ETSU ;
- SQUALANE AND VINYL DIMETHICONE/LAURYL DIMETHICONE CROSSPOLYMER as "KSG 44" sold by the company SHIN ETSU .

According to one embodiment, the cosmetic composition according to the present invention may be in the form of a powdery composition or a liquid or solid composition, such as an oily-solid cosmetic composition or an anhydrous composition.

In particular, the powdery cosmetic composition according to the present invention can have reduced friction which provides a smooth feeling to use, and can have good compactability which provides high stability against physical impact, due to the inclusion of the composite pigment or composite pigment composition according to the present invention.

Furthermore, the powdery cosmetic composition according to the present invention can show preferable cosmetic effects such as good fitting to the skin, homogeneous appearance, hiding the color of the skin, hiding the pores and lines on the skin, making the pores and lines on the skin less remarkable, and matt appearance, due to the inclusion of the composite pigment or composite pigment composition according to the present invention.

On the other hand, the liquid cosmetic composition according to the present invention can show good visual optical effects such as matt and haze effects, due to the inclusion of the composite pigment or composite pigment composition according to the present invention.

In any event, the powdery and liquid cosmetic composition according to the present invention has better UV filtering effects and/or better coloring effects, in addition to reduce the risk of fine particles of inorganic solid UV filter(s) and/or coloring pigment(s) penetrating into the skin via pores on the skin.

According to another embodiment, the cosmetic composition according to the present invention may be in the form of, for example, a compact powder, a lotion, a serum, a milk, a cream, a base foundation, an undercoat, a make-up base coat, a foundation, a face powder, cheek rouge, a lipstick, a lip cream, an eye shadow, an eyeliner, a loose powder, a concealer, a nail coat, mascara, a sunscreen and the like.

It is to be understood that a person skilled in the art can choose the appropriate presentation form, as well as its method of preparation, on the basis of his/her general knowledge, taking into account the nature of the constituents used, for example, their solubility in the vehicle, and the application envisaged for the composition.

The cosmetic compositions according to the invention may be also used, for example, as care products and/or antisun protection products for the face and/or the body, of liquid to semi-liquid consistency, such as milks, more or less rich creams, cream-gels and pastes. They may optionally be packaged as an aerosol and may be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and also aerosol pumps using compressed air as propellant. These devices are described in patents US 4 077 441 and US 4 850 517.

The compositions conditioned in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

The compositions according to the invention may additionally, further, comprise additional cosmetic and dermatological active agents.

EXAMPLES

The present invention will be described in more detail by way of examples, which however should not be construed as limiting the scope of the present invention.

**Examples 1 to 28 and Comparative Examples 1 to 7**

The components shown in Tables 1 to 4 were subjected to a hybridizer process using a Hybridizer equipped with a high speed rotor having a plurality of blades in a chamber in dry conditions, marketed by Nara Machinery Co., Ltd. in Japan to obtain a composite pigment.

In detail, for each of Examples 1-28 and Comparative Examples 1-7, the components shown in Tables 1 to 4 were mixed at the mixing ratio (the numerals in Tables 1 to 4 are based on parts by weight) shown in Tables 1 to 4 in a plastic bag by hand shaking for a short period of time. The mixture was put in the Hybridizer, and the rotor was revolved at 8,000 rpm (100 m/s linear velocity) for 3 minutes to obtain the composite pigments according to Examples 1-28 and Comparative Examples 1-7.

[UV Absorbance Evaluation]

Absorbance of UV waves of each of the composite pigments according to Examples 1-28 and Comparative Examples 1-7 was measured by use of a UV/VIS spectrophotometer type V-550 (JASCO, Japan) as follows.

A solvent was prepared by mixing isododecane and polyhydroxystearic acid such that the concentration of polyhydroxystearic acid was 3 wt%.

Each of the composite pigments according to Examples 1-28 and Comparative Examples 1-7 was dispersed in the above solvent by using ultrasonic waves for 1 minute to obtain a sample, such that the concentration of the composite pigment in the sample was 0.1 wt%. If agglomerates were still present, the ultrasonic treatment was repeated.

The obtained sample was put into a quartz cell having a 2 mm light pathway. The UV absorbance of the sample in the wavelength of from 280 to 400 nm was measured by use of a UV/VIS spectrophotometer type V-550 (JASCO, Japan).

The results are shown in Tables 1 to 4.

[Friction Evaluation]

The mean friction coefficient (MIU) of the composite pigments of Examples 18 and 26 as well as Comparative Example 1 was measured by a friction tester (KES-SE Friction Tester, Kato-Tech, Japan). As References 1 and 2, the mean friction coefficient of each of 3$TiO_2$(1)(MT-100 TV) particles and $TiO_2$(2)(SA-TTO-S4) particles was also measured.

Conditions for the MIU measurement were as follows.

Substrate: glass plate (5 cm*2 cm)

Probe: metal sensor (1.0 cm*1.0 cm; 25.0 g)
Applied Pressure: 2.45 kPa
Speed: 1 mm/sec

The results are shown in Table 5.

Table 1

| | Core | | | | | | UV filter | | UV* |
|---|---|---|---|---|---|---|---|---|---|
| | PMMA* | PMMA** | Nylon 12* | Nylon 12** | Silica | PTFE | $TiO_2$(1) | Mex-XL | |
| Particle Size | 350 nm | 150 nm | 5 μm | 10 μm | 3 μm | 3. 5 μm | 15 nm | - | |
| Ex. 1 | 90 | - | - | - | - | - | 10 | - | 24 |
| Ex. 2 | 70 | - | - | - | - | - | 20 | 10 | 72 |
| Ex. 3 | 70 | - | - | - | - | - | 30 | - | 72 |
| Ex. 4 | 50 | - | - | - | - | - | 50 | - | 139 |
| Ex. 5 | 30 | - | - | - | - | - | 70 | - | 189 |
| Ex. 6 | - | 70 | - | - | - | - | 30 | - | 101 |
| Comp. Ex. 1 | - | - | 70 | - | - | - | 30 | - | 14 |
| Comp. Ex. 2 | - | - | - | 70 | - | - | 30 | - | 4 |
| Comp. Ex. 3 | - | - | - | - | 70 | 70 | 30 | - | 11 |
| Comp. Ex. 4 | - | - | - | - | - | - | 30 | - | 20 |

PMMA*: MP-2200 marketed by Soken in Japan
PMMA**: MP-1451 marketed by Soken in Japan
Nylon 12*: SP-500 marketed by Toray in Japan
Nylon 12**: Orgasol marketed by Arkema in France
Silica: P1500 marketed by JGC C&C in Japan
PTFE: Ceridust 9205F marketed by Clariant in Switzerland
$TiO_2$(1): MT-100 TV marketed by Tayca in Japan
Mex-XL: Drometrizole trisiloxane marketed by Chimex in France
UV*: UV absorbance in the wavelength region from 280 to 400 nm

Table 2

| | Core | | | | UV filter | | UV* |
|---|---|---|---|---|---|---|---|
| | PMMA | Nylon 12* | Nylon 12** | Silica | $TiO_2$(2) | $TriO_2$(3) | |
| Particle Size | 350 nm | 5 μm | 10 μm | 3.5 μm | 15 nm | 20 nm | |
| Ex. 7 | 70 | - | - | - | 30 | - | 78 |
| Ex. 8 | 70 | - | - | - | - | 30 | 65 |
| Comp. Ex. 5 | - | 70 | - | - | 30 | - | 13 |
| Comp. Ex. 6 | - | - | 70 | - | - | 30 | 16 |
| Comp. Ex. 7 | - | - | - | 70 | - | 30 | 9 |

PMMA: MP-2200 marketed by Soken in Japan
Nylon 12*: SP-500 marketed by Toray in Japan
Nylon 12**: Orgasol marketed by Arkema in France
Silica: P1500 marketed by JCG C&C in Japan
$TiO_2$(2): SA-TTO-S4 marketed by Miyoshi Kasei in Japan
$TriO_2$(3): P-25 marketed by Degussa in Germany
UV*: UV absorbance in the wavelength region from 280 to 400 nm

Table 3

| | Core | UV filter | | | | | | | | |
|---|---|---|---|---|---|---|---|---|---|---|
| | PMMA | $TiO_2$ (4) | $TiO_2$ (5) | $TiO_2$ (6) | $TiO_2$ (7) | $TiO_2$ (8) | $TiO_2$ (9) | $TiO_2$ (10) | $TiO_2$ (11) | UV* |
| Particle Size | 350 nm | 10 nm | 10 nm | 15 nm | 15 nm | 15 nm | 15 nm | 15 nm | 15 nm | |
| EX. 9 | 70 | 30 | - | - | - | - | - | - | - | 65 |
| Ex. 10 | 70 | - | 30 | - | - | - | - | - | - | 56 |
| Ex. 11 | 70 | - | - | 30 | - | - | - | - | - | 59 |
| Ex. 12 | 70 | - | - | - | 30 | - | - | - | - | 62 |
| Ex. 13 | 70 | - | - | - | - | 30 | - | - | - | 65 |
| Ex. 14 | 70 | - | - | - | - | - | 30 | - | - | 58 |
| Ex. 15 | 70 | - | - | - | - | - | - | 30 | - | 33 |
| Ex. 16 | 70 | - | - | - | - | - | - | - | 30 | 138 |

PMMA: MP-2200 marketed by Soken in Japan
$TiO_2$(4): MT-Y02 marketed by Tayca in Japan
$TiO_2$(5): MT-Y-100M3S marketed by Tayca in Japan
$TiO_2$(6): MT-100WP marketed by Tayca in Japan
$TiO_2$(7): MT-100SA marketed by Tayca in Japan
$TiO_2$(8): SA-TTO-S3 marketed by Miyoshi Kasei in Japan
$TiO_2$(9): UV TITAN M170 marketed by Sachtleben in Germany
$TiO_2$(10): HT-100AQ marketed by Tayca in Japan
$TiO_2$(11): HXMT-100ZA marketed by Tayca in Japan
UV*: UV absorbance in the wavelength region from 280 to 400 nm

Table 4

| | Core | | | | | | UV filter | | | |
|---|---|---|---|---|---|---|---|---|---|---|
| | PMMA | Nylon 12* | Nylon 12** | Silica* | PTFE | Silica** | $TiO_2$ (1) | $TiO_2$ (2) | $TiO_2$ (11) | UV* |
| Particle Size | 350 nm | 5 μm | 10 | 3 μm | 3.5 μm | 5 μm | 10 nm | 15 nm | 15 nm | |
| Ex. 17 | 50 | 20 | - | - | - | | 30 | - | - | 91 |
| Ex. 18 | 35 | 35 | - | - | - | - | 30 | - | - | 72 |
| Ex. 19 | 35 | 30 | - | - | - | - | 35 | - | - | 79 |
| Ex. 20 | 35 | 15 | - | - | - | - | 50 | - | - | 148 |
| Ex. 21 | 35 | - | 35 | - | - | - | 30 | - | - | 87 |
| Ex. 22 | 35 | - | - | 35 | - | - | 30 | - | - | 70 |
| Ex. 23 | 35 | - | - | - | 35 | - | 30 | - | - | 68 |
| Ex. 24 | 50 | - | 20 | - | - | - | 30 | - | - | 105 |
| Ex. 25 | 50 | - | - | - | - | 20 | 30 | - | - | 96 |
| Ex. 26 | 35 | 35 | - | - | - | - | - | 30 | - | 105 |
| Ex. 27 | 35 | 15 | - | - | - | - | - | 50 | - | 177 |

(continued)

| | Core | | | | | | UV filter | | | UV* |
|---|---|---|---|---|---|---|---|---|---|---|
| | PMMA | Nylon 12* | Nylon 12** | Silica* | PTFE | Silica** | $TiO_2$ (1) | $TiO_2$ (2) | $TiO_2$ (11) | |
| Particle Size | 350 nm | 5 μm | 10 | 3 μm | 3.5 μm | 5 μm | 10 nm | 15 nm | 15 nm | |
| Ex. 28 | 35 | 35 | - | - | - | - | - | - | 30 | 125 |

PMMA: MP-2200 marketed by Soken in Japan
Nylon 12*: SP-500 marketed by Toray in Japan
Nylon 12**: Orgasol marketed by Arkema in France
Silica*: P1500 marketed by JGC C&C in Japan
PTFE: Ceridust 9205F marketed by Clariant in Switzerland
Silica**: ACEMATT OK 412 marketed by Degussa in Germany
$TiO_2$(1): MT-100 TV marketed by Tayca Corporation in Japan
$TiO_2$(2): SA-TTO-S4 marketed by Miyoshi Kasei in Japan
$TiO_2$(11): HXMT-100ZA marketed by Tayca in Japan
UV*: UV absorbance in the wavelength region from 280 to 400 nm

Table 5

| | Ratio (wt%) | | | | MIU | |
|---|---|---|---|---|---|---|
| | PMMA | Nylon 12* | $TiO_2$ (1) | $TiO_2$ (2) | Average | σ (95%) |
| Ex. 18 | 35 | 35 | 30 | - | 0.566 | 0.021 |
| Ex. 26 | 35 | 35 | - | 30 | 0.534 | 0.024 |
| Comp. Ex. 1 | - | 70 | 30 | - | 0.626 | 0.032 |
| Ref. 1 | - | - | 100 | - | 0.746 | 0.033 |
| Ref. 2 | - | - | - | 100 | 0.756 | 0.052 |

PMMA: MP-2200 marketed by Soken in Japan
Nylon 12*: SP-500 marketed by Toray in Japan
$TiO_2$(1): MT-100 TV marketed by Tayca in Japan
$TiO_2$(2): SA-TTO-S4 marketed by Miyoshi Kasei in Japan
σ: Standard deviation

It is clear from Table 1 that the composite pigments according to Examples 1-6 using a small single core particle have higher UV absorbance than the composite pigments according to Comparative Examples 1-4 using a large single core particle. Further, it is found from Examples 1-5 that the more the UV filter is used, the more UV absorbance is obtained. Furthermore, it is found from Examples 3 and 6 that the smaller the core particle, the higher the UV absorbance.

The same can be found in Table 2. The composite pigments according to Examples 7 and 8 using a small single core particle have higher UV absorbance than the composite pigments according to Comparative Examples 5-7 using a large single core particle.

Table 3 shows, in combination with Tables 1 and 2, that even though the type of the UV filters is different, the UV absorbance of the composite pigments according to Examples 9-16 is higher than those of Comparative Examples 1-7. It should be noted that the UV filter in which a core $TiO_2$ is coated with another UV filter (Avobenzone) gives very high UV absorbance (see Example 16).

Table 4 shows that when a small core particle is combined with a large particle, the obtained composite pigment composition can give better UV absorbance. It should be noted that the UV filter in which a core $TiO_2$ is coated with another UV filter (Avobenzone) gives very high UV absorbance (see Examples 18 and 28).

Accordingly, it is clear that the composite pigment compositions according to the present invention can provide a better UV absorbance.

Table 5 shows that the composite pigment compositions according to the present invention have reduced friction, as compared to the composite pigment according to Comparative Example 1 as well as the $TiO_2$ powders according to References 1 and 2.

### Examples 29 to 40 and Comparative Examples 8 to 10

A powdery foundation was prepared by mixing the composite pigment composition according to Examples 18, 20, 26 or 27, or Comparative Example 1 with a powdery foundation base having the formula shown in Table 6 to obtain the powdery foundation, such that the concentration of the composite pigment composition in the powdery foundation was 5 wt%, 10 wt% or 20 wt%. In other words, 5, 10 or 20 parts by weight of the composite pigment composition was mixed with 95, 90 or 80 parts by weight of the powdery foundation base.

Table 6

| Phase | Component | wt% |
|---|---|---|
| A | Talc | 39.8 |
| | Sericite | 35 |
| | Mica | 5 |
| | Zinc Stearate/Zinc Oxide | 1 |
| B1 | Titanium Oxide | 8 |
| B2 | Iron Oxide (Red) | 0.5 |
| | Iron Oxide (Yellow) | 1.5 |
| | Iron Oxide (Blue) | 0.2 |
| D | Mineral Oil/Paraffin Liquid | 4 |
| | Phenyltrimethicone | 4.5 |
| | Phenoxyethanol | 0.5 |

In other words, the components in Phases A, B1 and B2, as well as the composite pigment composition according to Examples 18, 20, 26 or 27, were mixed in a mixer. Next, the components in Phase D were added and mixed furthermore. Then, the mixture was ground and sifted to obtain a powdery foundation according to each of Examples 29 to 40, and Comparative Examples 8 to 10.

[Friction Evaluation]

The mean friction coefficients (MIU) of a powdery foundation comprising the composite pigment composition according to Example 18, 20, 26 or 27, and of another powdery foundation comprising the composite pigment according to Comparative Example 1, in the same amount of 5 wt%, 10 wt% or 20 wt%, were measured by a friction tester (KES-SE Friction Tester, Kato-Tech, Japan).

Conditions for the MIU measurement were as follows.
Substrate: 2 mm thick sheet (5 cm*2 cm)
Probe: 2 mm thick sheet (1.3 cm*1.2 cm)
Applied Pressure: 1.37 kPa
Speed: 1 mm/sec

The results are shown in Table 7.

[Compactability Evaluation]

A powdery foundation comprising the composite pigment composition according to Example 18, 20, 26 or 27, or the composite pigment according to Comparative Example 1, in an amount of 10 wt%, was subjected to the drop test in which each powdery foundation was dropped at the height of 20cm onto a ceramic tile. The time of drop which gave 5 wt% of loss was determined.

The results are shown in Table 8.

Table 7

| | Pigment | Ratio (wt%) | | | | MIU | | | | | |
|---|---|---|---|---|---|---|---|---|---|---|---|
| | | PMMA | Nylon 12* | $TiO_2$ (1) | $TiO_2$ (2) | 5 wt% | | 10 wt% | | 20 wt% | |
| | | | | | | Avg. | σ (95%) | Avg. | σ (95%) | Avg. | σ (95%) |
| Ex. 29 | Ex. 18 | 35 | 35 | 30 | - | 0.748 | 0.055 | - | - | - | - |
| Ex. 30 | Ex. 20 | 35 | 15 | 50 | - | 0.766 | 0.058 | - | - | - | - |
| Ex. 31 | Ex. 26 | 35 | 35 | - | 30 | 0.788 | 0.045 | - | - | - | - |
| Ex. 32 | Ex. 27 | 35 | 15 | - | 50 | 0.785 | 0.053 | - | - | - | - |
| Comp. Ex. 8 | Comp. Ex. 1 | - | 70 | 30 | - | 0.813 | 0.016 | - | - | - | - |
| Ex. 33 | Ex. 18 | 35 | 35 | 30 | - | - | - | 0.693 | 0.011 | - | - |
| Ex. 34 | Ex. 20 | 35 | 15 | 50 | - | - | - | 0.693 | 0.027 | - | - |
| Ex. 35 | Ex. 26 | 35 | 35 | - | 30 | - | - | 0.708 | 0.053 | - | - |
| Ex. 36 | Ex. 27 | 35 | 15 | - | 50 | - | - | 0.719 | 0.040 | - | - |
| Comp. Ex. 9 | Comp. Ex. 1 | - | 70 | 30 | - | - | - | 0.735 | 0.042 | - | - |
| Ex. 37 | Ex. 18 | 35 | 35 | 30 | - | - | - | - | - | 0.675 | 0.038 |
| Ex. 38 | Ex. 20 | 35 | 15 | 50 | - | - | - | - | - | 0.664 | 0.036 |
| Ex. 39 | Ex. 26 | 35 | 35 | - | 30 | - | - | - | - | 0.645 | 0.041 |
| Ex. 40 | Ex. 27 | 35 | 15 | - | 50 | - | - | - | - | 0.662 | 0.008 |
| Comp. Ex. 10 | Comp. Ex. 1 | - | 70 | 30 | - | - | - | - | - | 0.794 | 0.025 |

PMMA: MP-2200 marketed by Soken in Japan
Nylon 12*: SP-500 marketed by Toray in Japan
$TiO_2$(1): MT-100 TV marketed by Tayca in Japan
$TiO_2$(2): SA-TTO-S4 marketed by Miyoshi Kasei in Japan
σ: Standard deviation
Avg: Average

Table 8

| | Pigment | Ratio (wt%) | | | | Times | |
|---|---|---|---|---|---|---|---|
| | | PMMA | Nylon 12* | $TiO_2$ (1) | $TiO_2$ (2) | 10 wt% | |
| | | | | | | Avg. | σ (95%) |
| Ex. 33 | Ex. 18 | 35 | 35 | 30 | - | 34 | 1.8 |
| Ex. 34 | Ex. 20 | 35 | 15 | 50 | - | 56 | 5.6 |
| Ex. 35 | Ex. 26 | 35 | 35 | - | 30 | 45 | 5.4 |
| Ex. 36 | Ex. 27 | 35 | 15 | - | 50 | 97 | 6.2 |

(continued)

| | Pigment | Ratio (wt%) | | | | Times | |
|---|---|---|---|---|---|---|---|
| | | PMMA | Nylon 12* | $TiO_2$ (1) | $TiO_2$ (2) | 10 wt% | |
| | | | | | | Avg. | σ (95%) |
| Comp. Ex. 9 | Comp. Ex. 1 | - | 70 | 30 | - | 19 | 2.5 |
| PMMA: MP-2200 marketed by Soken in Japan<br>Nylon 12*: SP-500 marketed by Toray in Japan<br>$TiO_2$(1): MT-100 TV marketed by Tayca in Japan<br>$TiO_2$(2): SA-TTO-S4 marketed by Miyoshi Kasei in Japan<br>σ: Standard deviation<br>Avg: Average | | | | | | | |

It is clear from Table 7 that the powdery foundations according to Examples 29-32, 33-36 and 37-40 have smaller friction coefficients than those according to Comparative Examples 8, 9 and 10, respectively. Therefore, the powdery foundations according to Examples 29-32, 33-36 and 37-40 can provide a smoother feel on touch than the powdery foundations according to Comparative Examples 8, 9 and 10. This trend is maintained even though the concentration of the composite pigment in the powdery foundation changes.

Table 8 shows that the powdery foundations according to Examples 33-36 have better compactability than that according to Comparative Example 9, and therefore, the former can be more stable against impacts than the latter.

[Sensory Evaluation]

The powdery foundation according to Example 33 and a control powdery foundation which has the same composition as Example 33 except that a simple mixture of 50 wt% of PMMA (MP2200) and 50 wt% of Nylon-12 (SP500) was used instead of the composite pigment composition according to Example 18, were compared.

Each of the powdery foundation of Example 33 and the control powdery foundation, in an amount of 1 g, was applied to the skin of 11 panelists, and the cosmetic effects (ex: fitting to the skin, homogeneous appearance, hiding the color of the skin, hiding the pores and lines on the skin, making the pore and lines on the skin less remarkable, and matt appearance) of each were evaluated and compared.

Specifically, the powdery foundation according to Example 33 was applied to half of the face, and the control powdery foundation was applied to the other half of the face. The cosmetic effects of the former were compared to those of the latter. The evaluations by 11 panelists were averaged.

The averaged results show that the powdery foundation according to Example 33 showed better cosmetic effects in terms of fitting to the skin, homogeneous appearance, hiding the color of the skin, hiding the pores and lines on the skin, making the pore and lines on the skin less remarkable, and matt appearance than the control powdery foundation.

**Examples 41 and 42, Comparative Example 11, and Reference 3**

A make-up base in the form of a W/O emulsion was prepared by mixing the components shown in Table 9.

Table 9

| Phase | Component | wt% |
|---|---|---|
| A1 | PEG-10 dimethicone | 2.0 |
| | BIS-PEG/PPG-14/14 dimethicone-dyclopentasiloxane | 1.0 |
| | Cyclopentasiloxane | 14.5 |
| | Dimethicone | 1.0 |
| | Ethylhexylmethoxycinnamate | 2.5 |
| | Tribehenin | 1.0 |
| | Dimethicone-dimethicone crosspolymer | 8.3 |

(continued)

| Phase | Component | wt% |
|---|---|---|
| A2 | Composite pigment or composite pigment composition | 13.0 |
| | Talc | 1.0 |
| B | Water | 46.0 |
| | Magnesium sulfate | 0.7 |
| | Methylparaben | 0.25 |
| | Phenoxyethanol | 0.5 |
| | Glycerin | 2.75 |
| | Butyleneglycol | 3.5 |
| | Maltitol-sorbitol | 1.0 |
| C | Ethanol | 1.0 |

The components in Phase A1 other than dimethicone-dimethicone crosspolymer were mixed in a container with a magnetic stirrer at 60°C. Next, dimethicone-dimethicone crosspolymer was added and mixed furthermore. Then, the components in Phase A2 were added to the mixture, and stirred. Next, the components in Phase B were added to the mixture, and stirred. Lastly, the component in Phase C was added to the mixture, and homogenized to obtain a make-up base according to Example 41, Example 42 or Comparative Example 11.

As the composite pigment or composite pigment composition in Phase A2, the composite pigment according to Comparative Example 1 or the composite pigment composition according to Example 18 or 26 was used.

For the following evaluation of the matt and haze effects, as Reference 3, a make-up base having the above composition shown in Table 9 except that the composite pigment composition according to Example 18 or 26 was replaced with a simple mixture of 50 w% of PMMA (MP2200) and 50 wt% of Nylon-12 (SP500) was used.

[*In Vitro* SPF Value Evaluation]

The make-up base was applied onto a PMMA plate in an amount of 1 mg/cm$^2$, and the SPF value of the make-up base sample was measured by an SPF analyzer UV-2000S. The results are shown in Table 10.

[Matt Effect Evaluation]

The make-up base was applied onto the white shiny part of a contrast card to form a film with a thickness of 50 $\mu$m, and dried at room temperature for 24 hours. By using a goniophotometer, calibrations and light intensity measurements were performed respectively with a matt standard plate made from barium sulfate and with the contrast card coated with the make-up base. The configuration of the contrast card with the make-up base and the goniophotometer is shown in Figure 1. The matt effect was determined by the following formula.

$$\text{(Matt Effect)(\%)} = \{(I_{45}/I_0)\text{standard}/(I_{45}/I_0)\text{sample}\}*100$$

$I_{45}$: Light Intensity of Specular Reflectance
$I_0$: Light Intensity of Diffuse Reflectance

The results are shown in Table 11.

[Haze Effect Evaluation]

The make-up base was applied onto a quartz plate to form a film with a thickness of 20 $\mu$m, and dried at about 37°C for 10 minutes. The diffuse transmittance through the plate was measured with a spectrophotometer equipped with an integration sphere. The configuration of the plate with the make-up base and the spectrophotometer is shown in Figure 2. The haze effect was determined by the following formula.

$$(Haze\ Effect)(\%) = \{T(diffuse)/T(total)\}*100$$

T(diffuse): Transmittance of Diffused Light
T(total): Sum of Transmittance of Diffused Light and Transmittance of Direct Transmitted Light

The results are shown in Table 11.

Table 10

| | Pigment | Ratio (wt%) | | | | SPF Value | |
|---|---|---|---|---|---|---|---|
| | | PMMA | Nylon 12* | $TiO_2$ (1) | $TiO_2$ (2) | Avg. | σ (95%) |
| Ex. 41 | Ex. 18 | 35 | 35 | 30 | - | 28.0 | 0.65 |
| Ex. 42 | Ex. 26 | 35 | 35 | - | 30 | 29.5 | 2.71 |
| Comp. Ex. 11 | Comp. Ex. 1 | - | 70 | 30 | - | 9.2 | 2.48 |

PMMA: MP-2200 marketed by Soken in Japan
Nylon 12*: SP-500 marketed by Toray in Japan
$TiO_2$(1): MT-100 TV marketed by Tayca in Japan
$TiO_2$(2): SA-TTO-S4 marketed by Miyoshi Kasei in Japan
σ: Standard deviation
Avg: Average

Table 11

| | Pigment | Ratio (wt%) | | | | Matt Effect | | Haze Effect | |
|---|---|---|---|---|---|---|---|---|---|
| | | PMMA | Nylon 12* | $TiO_2$ (1) | $TiO_2$ (2) | Avg. | σ (95%) | Avg. | σ (95%) |
| Ex. 41 | Ex. 18 | 35 | 35 | 30 | - | 59.8 | 1.1 | 85.4 | 3.0 |
| Ex. 42 | Ex. 26 | 35 | 35 | - | 30 | 52.8 | 0.9 | 81.3 | 2.2 |
| Ref. 3 | | 50 | 50 | - | - | 40.0 | 1.7 | 64.6 | 2.7 |

PMMA: MP-2200 marketed by Soken in Japan
Nylon 12*: SP-500 marketed by Toray in Japan
$TiO_2$(1): MT-100 TV marketed by Tayca in Japan
$TiO_2$(2): SA-TTO-S4 marketed by Miyoshi Kasei in Japan
σ: Standard deviation
Avg: Average

Table 10 shows that the make-up bases according to Examples 41 and 42 have better SPF values than that according to Comparative Example 11, and therefore, the former can provide better UV protection.

It is clear from Table 11 that the make-up bases according to Examples 41 and 42 have better matt and haze effects than that according to Reference 3. Therefore, the make-up bases according to the present invention can control excess gloss on the skin.

**Example 43**

The composite pigment or the composite pigment composition according to the present invention has advantageous UV filtering effects, and therefore, it can be incorporated into UV shielding products such as a sun care emulsion. An example (Example 43) of the sun care emulsion is shown in Table 12.

Table 12

| Phase | Component | wt% |
|---|---|---|
| A | Water | 50.6 |
| | Methylparaben | 0.2 |
| | Glycerin | 7.0 |
| | Glycol | 6.0 |
| | Terephthalylidene dicamphor sulfonic acid | 1.0 |
| | Triethanolamine | 0.3 |
| B | $C_{12}$-$C_{15}$ alkylbenzoate | 5.5 |
| | Octocrylene | 5.0 |
| | Butyl methoxydibenzoylmethane | 2.0 |
| | Ethylhexyl triazone | 0.5 |
| | Stearic acid | 1.0 |
| | Triethanolamine | 0.3 |
| | Glyceryl stearate (and) PEG-100 stearate | 2.0 |
| | Polydimethylsiloxane | 0.6 |
| | Ethylparaben | 0.3 |
| | Phenoxyethanol | 0.7 |
| | Isohexadecane | 5.0 |
| | Stearyl alcohol | 2.0 |
| C | Composite pigment or composite pigment composition | 10.0 |

The sun care emulsion according to Example 43 can be prepared by mixing the components in Phase A at 60°C, further mixing the components in Phase B at 60°C, furthermore homogenizing the mixture of the components in Phases A and B in a Homomixer, followed by adding the component in Phase C to the obtained mixture and stirring well.

<u>Example 44 and 45 and comparative examples 46 to 48: in vitro SPF measurement for sun-care formulas</u>

[Preparation of the compositions]

10% (ex. 44) and 5% (ex. 45) of the composite pigment composition were introduced into sun-care formulas (O/W emulsion) prepared according to protocol disclosed in example 43.

Comparative formulas were prepared but with mixture of powders instead of the composite pigment composition (comparative ex. 46 and 47) or without any powders nor any composite pigment(comparative ex. 48= base formula alone).

[Method of in vitro SPF measurement]

The sun protection factor (SPF) is determined according to the "*in* vitro" method described by B.L. Diffey in J. Soc. Cosmet. Chem. 40, 127-133, (1989). The measurements were made using a UV-2000S spectrophotometer from the company Labsphere. Each composition is applied to a rough plate of PMMA, in the form of a homogeneous and even deposit at a rate of 0.75mg/$cm^2$.

The table 13 below describes the in vitro SPF value corresponding of the each formula.

Table 13

| Phase | | Ex. 44 (invention) | Ex. 46 (comparative ) | Ex. 45 (invention) | Ex. 47 (comparative ) | Ex. 48 (comparative ) |
|---|---|---|---|---|---|---|
| | **SPF value** | **93** | **69** | **76** | **56** | **7** |
| | **INCI US Name** | Wt% | Wt% | Wt% | Wt% | Wt% |
| A | WATER | 35 | 35 | 35 | 35 | 35 |
| | METHYLPARABEN | 0.4 | 0.4 | 0.4 | 0.4 | 0.4 |
| | GLYCERIN | 6 | 6 | 6 | 6 | 6 |
| | PROPYLENE GLYCOL | 6 | 6 | 6 | 6 | 6 |
| | TEREPHTHALYLIDENE DICAMPHOR SULFONIC ACID | 4.5 | 4.5 | 4.5 | 4.5 | 4.5 |
| | TRIETHANOLAMINE | 0.16 | 0.16 | 0.16 | 0.16 | 0.16 |
| | DISODIUM EDTA | 0.1 | 0.1 | 0.1 | 0.1 | 0.1 |
| | POTASSIUM CETYL PHOSPHATE | 1 | 1 | 1 | 1 | 1 |
| B1 | C12-15 ALKYL BENZOATE | 7.5 | 7.5 | 7.5 | 7.5 | 7.5 |
| | BUTYL METHOXYDIBENZOYLM ETHANE | 2 | 2 | 2 | 2 | 2 |
| | N-HEXYL 2-(4-DIETHYLAMINO-2-HYDROXYBENZOYL)BEN ZOATE(UVINUL A+) | 4 | 4 | 4 | 4 | 4 |
| | ETHYLHEXYL TRIAZONE (UVINUL T150) | 2 | 2 | 2 | 2 | 2 |
| | STEARIC ACID | 1 | 1 | 1 | 1 | 1 |
| | TRIETHANOLAMINE | 0.3 | 0.3 | 0.3 | 0.3 | 0.3 |
| | GLYCERYL STEARATE (and) PEG-100 STEARATE | 1 | 1 | 1 | 1 | 1 |
| | DIMETHICONE | 0.5 | 0.5 | 0.5 | 0.5 | 0.5 |
| | ETHYLPARABEN | 0.3 | 0.3 | 0.3 | 0.3 | 0.3 |
| | TOCOPHEROL | 0.1 | 0.1 | 0.1 | 0.1 | 0.1 |
| | PHENOXYETHANOL | 0.7 | 0.7 | 0.7 | 0.7 | 0.7 |

(continued)

| Phase | | Ex. 44 (invention) | Ex. 46 (comparative ) | Ex. 45 (invention) | Ex. 47 (comparative ) | Ex. 48 (comparative ) |
|---|---|---|---|---|---|---|
| B3 | **COMPOSITE PIGMENT COMPOSITION OF EX. 18** | **10** | **-** | **-** | **-** | **-** |
| | **COMPOSITE PIGMENT COMPOSITION OF EXAMPLE 20** | **-** | **-** | **5** | **-** | **-** |
| | PMMA (MP-2200) | - | 3.5 | - | 1.75 | - |
| | Nylon-12 (SP-500) | - | - | - | 0.75 | - |
| | TITANIUM DIOXIDE (and) ALUMINUM HYDROXIDE (and) STEARIC ACID | - | 3 | - | 2.5 | - |
| C | ISOHEXADECANE | 4.5 | 4.5 | 4.5 | 4.5 | 4.5 |
| | ACRYLATES/C10-30 ALKYL ACRYLATE CROSSPOLYMER | 0.25 | 0.25 | 0.25 | 0.25 | 0.25 |
| | XANTHAN GUM | 0.1 | 0.1 | 0.1 | 0.1 | 0.1 |
| D | WATER | 1 | 1 | 1 | 1 | 1 |
| | TRIETHANOLAMINE | 0.25 | 0.25 | 0.25 | 0.25 | 0.25 |
| E | WATER | 9.34 | 9.34 | 14.34 | 14.34 | 19.34 |
| F | ALCOHOL DENT. | 2 | 2 | 2 | 2 | 2 |
| **Total (%):** | | 100 | 100 | 100 | 100 | 100 |

The base formula contains totally 12.5% of liquid organic UV filters, but in case of the base formula alone (comparative ex. 48), it is not effective (SPF value = 7). The UV protection effect was improved in presence of the mixture of powders (comparative ex. 46 and comparative ex. 47), and is even better with the composite pigment composition (ex. 44 and ex. 45) in comparison to the mixture of powders.

Examples 49 and 50: Sun care formulas

Table 14

| | | Example 49 | Example 50 |
|---|---|---|---|
| | **INCI US Name** | Wt % | Wt % |
| **A** | GLYCERIN | 5 | 5 |
| | EDTA | 0,1 | 0,1 |
| | POTASSIUM CETYL PHOSPHATE (AMPHISOL K) | 1 | 1 |
| | TEREPHTHALYLIDENE DICAMPHOR SULFONIC ACID (MEXORYL SX) | 1% (active material) | 1% (active material) |
| | DEIONIZED WATER | Qsp 100 | Qsp 100 |
| | TRIETHANOLAMINE | 0,3 | 0,3 |
| | PRESERVATIVES | 1,2 | 1,2 |
| | | | |
| **B1** | C12-15 ALKYL BENZOATE (TEGOSOFT TN) | 20 | 20 |
| | PRESERVATIVES | 0,25 | 0,25 |
| | STEARIC ACID | 1,5 | 1,5 |
| | GLYCERYL STEARATE (and) PEG-100 STEARATE | 1 | 1 |
| | CETYL ALCOHOL | 0,5 | 0,5 |
| | STEARYL ALCOHOL and STEARYL GLUCOSIDE | 2 | 2 |
| | POLY DIMETHYLSILOXANE (350 cst) | 0,5 | 0,5 |
| | TRIETHANOLAMINE | 0,45 | 0,45 |
| | BUTYLMETHOXYDIBENZOYLMETHANE (PARSOL 1789 -DSM) | 4 | 4 |
| | **COMPOSITE PIGMENT COMPOSITION OF EXAMPLE 18** | 4 | 0 |
| | **COMPOSITE PIGMENT POMPOSITION OF EXAMPLE 20** | 0 | 8 |
| | DROMETRIZOLE TRISILOXANE (MEXORYL XL) | 3 | 3 |
| | 2-ETHYLHEXYL a -CYANO-β,β'-DIPHENYLACRYLATE | 5 | 5 |
| | ETHYLHEXYL TRIAZONE (UVINUL T150) | 1 | 1 |
| | 2,4,6-TRIS[P-(2'-ÉTHYLHEXYL-1'-OXY-CARBONYL)ANILINO]-1,3,5-TRIAZINE | 1 | 1 |
| | TITANIUM DIOXIDE (AND) ALUMINUM HYDROXIDE (AND) STEARIC ACID (MT TV) | 5 | 5 |
| | | | |
| **B2** | ISOHEXADECANE | 1 | 1 |
| | ACRYLATES/C10-30 ALKYL ACRYLATE CROSSPOLYMER | 0,2 | 0,2 |
| | XANTHAN GUM | 0,21 | 0,2 |
| | CYCLOPENTADIMETHYLSILOXANE | 5 | 5 |

The aqueous phase A and oily phase $B_1$ are prepared by mixing the raw materials, with mechanical stirring, at 80°C; the solutions obtained are macroscopically homogeneous. The emulsion is prepared by slow introduction of the oily phase into the aqueous phase with stirring using a Moritz homogenizer at a stirring speed of 4000 rpm for 15 minutes. The emulsion obtained is cooled, with stirring, to 40°C, then the oily phase $B_2$ is added thereto with gentle stirring, followed by the phase C and phase D. The emulsion obtained is cooled to room temperature with gentle stirring. It is characterized by drops between 1 μm and 10 μm in size.

The compositions confer good sun protection.

Example 51: Skin care formula

A skin care formula was prepared by mixing the components shown in Table 15 according to common knowledge for cosmetic formulation.

Table 15

| Cosmetic type | INCI US | % by weight |
|---|---|---|
| SURFACTANT | STEARIC ACID | 0.1 |
| SURFACTANT | POTASSIUM CETYL PHOSPHATE | 0.05 |
| SURFACTANT | SUCROSE STEARATE | 3 |
| **COMPOSITE PIGMENT** | **COMPOSITE PIGMENT COMPOSITON OF EXAMPLE 20** | **15** |
| FILLER | **VINYL DIMETHICONE/METHICONE SILSESQUIOXANE** CROSSPOLYMER | 2 |
| FILLER | BORON NITRIDE | 1 |
| FILLER | SILICA | 1 |
| FATTY COMPOUND | CAPRYLIC/CAPRIC TRIGLYCERIDE | 4.5 |
| FATTY COMPOUND | HYDROGENATED LECIHIN | 0.3 |
| FATTY COMPOUND | DICAPRYLYL CARBONATE | 2 |
| POLYMER | XANTHAN GUM | 0.25 |
| POLYMER | CARBOMER | 0.2 |
| POLYMER | ACRYLATES/C10-30 ALKYL ACRYLATE CROSSPOLYMER | 0.1 |
| SOLVENT | ALCOHOL DENAT. | 3 |
| SOLVENT | WATER | Qsp 100 |
| SOLVENT | GLYCERIN | 7 |
| SOLVENT | CAPRYLYL GLYCOL | 0.3 |
| PRESERVATIVE | SALICYLIC ACID | 0.2 |
| PRESERVATIVE | PHENOXYETHANOL | 0.3 |

The skin care composition presents a good sun protection.

## Claims

1. A composite pigment comprising
at least one small particle with a mean particle size of more than 100 nm and less than 1 μm, preferably less than 600 nm, and more preferably less than 400 nm, wherein the surface of the small particle is at least in part covered with at least one layer comprising at least one inorganic solid UV filter particle.

2. The composite pigment according to Claim 1, wherein the inorganic solid UV filter is selected from the group consisting of silicon carbide, metal oxides, and mixtures thereof.

3. The composite pigment according to Claim 1 or 2, wherein the inorganic solid UV filter has a mean particle size of 1 nm to 50 nm, preferably 5 nm to 40 nm, and more preferably 10 nm to 30 nm.

4. The composite pigment according to any one of Claims 1 to 3, wherein the inorganic solid UV filter has at least one coating.

5. The composite pigment according to Claim 4, wherein the coating of the inorganic solid UV filter comprises at least one compound selected from the group consisting of alumina, silica, aluminum hydroxide, silicones, silanes, fatty acids or salts thereof, fatty alcohols, lecithin, amino acids, polysaccharides, proteins, alkanolamines, waxes, (meth)acrylic polymers, organic UV filters, and (per)fluoro compounds.

6. The composite pigment according to any one of Claims 1 to 5, wherein the at least one layer has a thickness of 1 nm to 50 nm, preferably 5 nm to 40 nm, and more preferably 10 nm to 30 nm.

7. The composite pigment according to any one of Claims 1 to 6, wherein the at least one layer further comprises at least one additional UV filter, in particular at least one organic UV filter.

8. The composite pigment according to any one of Claims 1 to 7, wherein the small particle comprises at least one inorganic material and/or at least one organic material, preferably at least one organic material.

9. The composite pigment according to Claim 8, wherein the inorganic material is selected from the group consisting of mica, synthetic mica, talc, sericite, boron nitride, glass flake, calcium carbonate, barium sulfate, titanium oxide, hydroxyapatite, silica, silicate, zinc oxide, magnesium sulfate, magnesium carbonate, magnesium trisilicate, aluminum oxide, aluminum silicate, calcium silicate, calcium phosphate, magnesium oxide, bismuth oxychloride, kaolin, hydrotalcite, mineral clay, synthetic clay, iron oxide, and mixtures thereof.

10. The composite pigment according to Claim 8, wherein the organic material is selected from the group consisting of poly(meth)acrylates, polyamides, silicones, polyurethanes, polyethylenes, polypropylenes, polystyrenes, polyhydroxyalkanoates, polycaprolactams, poly(butylene) succinates, polysaccharides, polypeptides, polyvinyl alcohols, polyvinyl resins, fluoropolymers, wax, amidosulfonic acid polyvalent metal salts, acylated amino acids, and mixtures thereof.

11. The composite pigment according to any one of Claims 1 to 10, wherein the weight ratio of the small particle(s) to the inorganic solid UV filter(s) and/or the coloring pigment(s) is 50:50 to 90:10, preferably 50:50 to 80:20, and more preferably 50:50 to 70:30.

12. A method for preparing a composite pigment, comprising a step of subjecting:

at least one small particle with a mean particle size of more than 100 nm and less than 1 μm, preferably less than 600 nm, and more preferably less than 400 nm;
at least one inorganic solid UV filter particle and/or at least one coloring pigment; and
optionally at least one additional UV filter,
to a mechanochemical fusion process.

13. A composite pigment composition, comprising:

at least one composite pigment according to any one of Claims 1 to 12; and
at least one large particle with a mean particle size of 2 μm or more, preferably 3 μm or more, more preferably 4 μm or more, and even more preferably 5 μm or more, wherein the surface of the large particle is optionally at least in part covered with at least one layer comprising at least one inorganic solid UV filter particle and/or at least one coloring pigment.

14. The composite pigment composition according to Claim 13, wherein the large particle comprises at least one inorganic material and/or at least one organic material, preferably at least one organic material.

**15.** The composite pigment composition according to Claim 13 or 14, wherein the weight ratio of the small particle(s) to the large particle(s) is 10:90 to 90:10, preferably 20:80 to 80:20, and more preferably 30:70 to 70:30.

**16.** The composite pigment composition according to any one of Claims 13 to 15, wherein the weight ratio of the small particle(s)/the large particle(s)/the inorganic solid UV filter particle(s) and/or the coloring pigment(s) is 20:50:30 to 50:20:30, preferably 35:15:50 to 15:35:50, and more preferably 10:20:70 to 20:10:70.

**17.** A method for preparing a composite pigment composition, comprising a step of subjecting:

at least one small particle with a mean particle size more than 100 nm and of less than 1 μm, preferably less than 600 nm, and more preferably less than 400 nm;
at least one inorganic solid UV filter particle and/or at least one coloring pigment;
at least one large particle with a mean particle size of 2 μm or more, preferably 3 μm or more, more preferably 4 μm or more, and even more preferably 5 μm or more; and
optionally at least one additional UV filter,
to a mechanochemical fusion process.

**18.** A cosmetic composition, in particular in the form of a liquid or a powdery cosmetic composition, comprising a composite pigment according to any one of Claims 1 to 11 or a composite pigment composition according to any one of Claims 13 to 16.

## Patentansprüche

**1.** Verbundpigment, umfassend
wenigstens einen kleinen Partikel mit einer durchschnittlichen Partikelgröße von mehr als 100 nm und weniger als 1μ, bevorzugt von weniger als 600 nm und eher bevorzugt von weniger als 400 nm, wobei die Oberfläche des kleinen Partikels wenigstens wenigstens teilweise mit einer Schicht bedeckt ist, die wenigstens einen anorganischen UV-Feststofffilterpartikel umfasst.

**2.** Verbundpigment nach Anspruch 1, wobei der anorganische UV-Feststofffilter ausgewählt ist aus der Gruppe bestehend aus Siliziumkarbid, Metalloxiden sowie Mischungen davon.

**3.** Verbundpigment nach Anspruch 1 oder 2, wobei der anorganische UV-Feststofffilter eine durchschnittliche Partikelgröße von 1 nm bis 50 nm, bevorzugt von 5 nm bis 40 nm und eher bevorzugt von 10 nm bis 30 nm aufweist.

**4.** Verbundpigment nach einem der Ansprüche 1 bis 3, wobei der anorganische UV-Feststofffilter wenigstens eine Beschichtung aufweist.

**5.** Verbundpigment nach Anspruch 4, wobei die Beschichtung des anorganischen UV-Feststofffilters wenigstens eine Verbindung umfasst, die ausgewählt ist aus der Gruppe bestehend aus Aluminiumdioxid, Siliziumdioxid, Aluminiumhydroxid, Silikonen, Silanen, Fettsäuren oder den Salzen davon, Fettalkoholen, Lezithin, Aminosäuren, Polysacchariden, Proteinen, Alkanolaminen, Wachsen, (Meth)Acrylpolymeren, organischen UV-Filtern und (Per)Fluorverbindungen.

**6.** Verbundpigment nach einem der Ansprüche 1 bis 5, wobei wenigstens eine Schicht eine Dicke von 1 nm bis 50 nm, bevorzugt 5 nm bis 40 nm und eher bevorzugt 10 nm bis 30 nm aufweist.

**7.** Verbundpigment nach einem der Ansprüche 1 bis 6, wobei die wenigstens eine Schicht ferner wenigstens einen zusätzlichen UV-Filter, insbesondere wenigstens einen organischen UV-Filter umfasst.

**8.** Verbundpigment nach einem der Ansprüche 1 bis 7, wobei der kleine Partikel wenigstens ein anorganisches Material und/oder wenigstens ein organisches Material, bevorzugt wenigstens ein organisches Material, umfasst.

**9.** Verbundpigment nach Anspruch 8, wobei das anorganische Material ausgewählt ist aus der Gruppe bestehend aus Glimmer, synthetischem Glimmer, Talk, Serizit, Bornitrid, Glasflocken, Kalziumcarbonat, Bariumsulfat, Titanoxid, Hydroxyapatit, Silizium, Silikat, Zinkoxid, Magnesiumsulfat, Magnesiumcarbonat, Magnesiumtrisilikat, Aluminiumoxid, Aluminiumsilikat, Kalziumsilikat, Kalziumphosphat, Magnesiumoxid, Bismutoxychlor, Kaolin, Hydrotalzid, Mineralton, synthetischem Ton, Eisenoxid und Mischungen davon.

**10.** Verbundpigment nach Anspruch 8, wobei das organische Material ausgewählt ist aus der Gruppe bestehend aus Poly(meth)acrylaten, Polymiden, Silikonen, Polyurethanen, Polyethylenen, Polypropylenen, Polystyrolen, Polyhydroxyalkanoaten, Polycaprolaktamen, Poly(butylen)succinaten, Polysacchariden, Polypeptiden, Polyvinylalkoholen, Polyvinylharzen, Fluorpolymeren, Wachs, Amidsulfonsäure, mehrwertigen Metallsalzen, acylierten Aminosäuren und Mischungen davon.

**11.** Verbundpigment nach einem der Ansprüche 1 bis 10, wobei das Gewichtsverhältnis der/des kleinen Partikel(s) zu dem/den anorganischen UV-Feststofffilter(n) und/oder dem/den Farbpigment(en) 50:50 bis 90:10, bevorzugt 50:50 bis 80:20 und eher bevorzugt 50:50 bis 70:30 beträgt.

**12.** Verfahren zur Herstellung eines Verbundpigments, umfassend einen Schritt des Aussetzens:

wenigstens eines kleinen Partikels mit einer durchschnittlichen Partikelgröße von mehr als 100 nm und weniger als 1 μm, bevorzugt von weniger als 600 nm und eher bevorzugt von weniger als 400 nm,
und
optional wenigstens eines zusätzlichen UV-Filters,
an ein mechanisch-chemisches Schmelzverfahren.

**13.** Verbundpigmentzusammensetzung, umfassend:

wenigstens ein Verbundpigment nach einem der Ansprüche 1 bis 12; und wenigstens einen großen Partikel mit einer durchschnittlichen Partikelgröße von 2 μm oder mehr, bevorzugt 3 μm oder mehr, eher bevorzugt 4 μm oder mehr und sogar noch eher bevorzugt 5 μm oder mehr, wobei die Oberfläche des großen Partikels optional wenigstens teilweise mit wenigstens einer Schicht bedeckt ist, die wenigstens einen anorganischen UV-Feststofffilterpartikel und/oder wenigstens ein Farbpigment umfasst.

**14.** Verbundpigmentzusammensetzung nach Anspruch 13, wobei der große Partikel wenigstens ein anorganisches Material und/oder wenigstens ein organisches Material, bevorzugt wenigstens ein organisches Material, umfasst.

**15.** Verbundpigmentzusammensetzung nach Anspruch 13 oder 14, wobei das Gewichtsverhältnis der/des kleinen Partikel(s) zu dem/den großen Partikel(n) 10:90 bis 90:10, bevorzugt 20:80 bis 80:20 und eher bevorzugt 30:70 bis 70:30 beträgt.

**16.** Verbundpigmentzusammensetzung nach einem der Ansprüche 13 bis 15, wobei das Gewichtsverhältnis der/des kleinen Partikel(s)/der des großen Partikel(s)/der/des anorganischen UV-Feststofffilterpartikel(s) und/oder des/der Farbpigment(e) 20:50:30 bis 50:20:30, bevorzugt 35:15:50 bis 15:35:50 und eher bevorzugt 10:20:70 bis 20:10:70 beträgt.

**17.** Verfahren zur Herstellung einer Verbundpigmentzusammensetzung, umfassend einen Schritt des Aussetzens:

wenigstens einen kleinen Partikel mit einer durchschnittlichen Partikelgröße von mehr als 100 nm und weniger als 1μm, bevorzugt von weniger als 600 nm und eher bevorzugt von weniger als 400 nm;
wenigstens eines anorganischen UV-Feststofffilterpartikels und/oder wenigstens eines Farbpigments;
wenigstens eines großen Partikels mit einer durchschnittlichen Partikelgröße von 2 μm oder mehr, bevorzugt 3 μm oder mehr, eher bevorzugt 4 μm oder mehr und sogar noch eher bevorzugt 5 μm oder mehr;
und
optional wenigstens eines zusätzlichen UV-Filters,
an ein mechanisch-chemisches Schmelzverfahren.

**18.** Kosmetische Zusammensetzung, insbesondere in der Form einer Flüssigkeit oder einer kosmetischen Pulverzusammensetzung, umfassend ein Verbundpigment nach einem der Ansprüche 1 bis 11 oder eine Verbundpigmentzusammensetzung nach einem der Ansprüche 13 bis 16.

## Revendications

1. Pigment composite comprenant
au moins une petite particule ayant une taille moyenne de particule supérieure à 100 nm et inférieure à 1 $\mu$m, de préférence inférieure à 600 nm, et plus préférablement inférieure à 400 nm,
dans lequel la surface de la petite particule est au moins en partie recouverte d'au moins une couche comprenant au moins une particule de filtre UV solide inorganique.

2. Pigment composite selon la revendication 1, dans lequel le filtre UV solide inorganique est choisi dans le groupe constitué du carbure de silicium, des oxydes de métaux et des mélanges de ceux-ci.

3. Pigment composite selon la revendication 1 ou 2, dans lequel le filtre UV solide inorganique a une taille moyenne de particule de 1 nm à 50 nm, de préférence de 5 nm à 40 nm, et plus préférablement de 10 nm à 30 nm.

4. Pigment composite selon l'une quelconque des revendications 1 à 3, dans lequel le filtre UV solide inorganique a au moins un revêtement.

5. Pigment composite selon la revendication 4, dans lequel le revêtement du filtre UV solide inorganique comprend au moins un composé choisi dans le groupe constitué de l'alumine, de la silice, de l'hydroxyde d'aluminium, des silicones, des silanes, des acides gras ou des sels de ceux-ci, des alcools gras, de la lécithine, des acides aminés, des polysaccharides, des protéines, des alcanolamines, des cires, des polymères (méth)acryliques, des filtres UV organiques et des composés (per)fluorés.

6. Pigment composite selon l'une quelconque des revendications 1 à 5, dans lequel l'au moins une couche a une épaisseur de 1 nm à 50 nm, de préférence de 5 nm à 40 nm et plus préférablement de 10 nm à 30 nm.

7. Pigment composite selon l'une quelconque des revendications 1 à 6, dans lequel l'au moins une couche comprend en outre au moins un filtre UV additionnel, en particulier au moins un filtre UV organique.

8. Pigment composite selon l'une quelconque des revendications 1 à 7, dans lequel la petite particule comprend au moins un matériau inorganique et/ou au moins un matériau organique, de préférence au moins un matériau organique.

9. Pigment composite selon la revendication 8, dans lequel le matériau inorganique est choisi dans le groupe constitué du mica, du mica synthétique, du talc, de la séricite, du nitrure de bore, du flocon de verre, du carbonate de calcium, du sulfate de baryum, de l'oxyde de titane, de l'hydroxyapatite, de la silice, du silicate, de l'oxyde de zinc, du sulfate de magnésium, du carbonate de magnésium, du trisilicate de magnésium, de l'oxyde d'aluminium, du silicate d'aluminium, du silicate de calcium, du phosphate de calcium, de l'oxyde de magnésium, de l'oxychlorure de bismuth, du kaolin, de l'hydrotalcite, de l'argile minérale, de l'argile synthétique, de l'oxyde de fer, et des mélanges de ceux-ci.

10. Pigment composite selon la revendication 8, dans lequel le matériau organique est choisi dans le groupe constitué des poly(méth)acrylates, des polyamides, des silicones, des polyuréthanes, des polyéthylènes, des polypropylènes, des polystyrènes, des polyhydroxyalcanoates, des polycaprolactames, des succinates de poly(butylène), des polysaccharides, des polypeptides, des alcools polyvinyliques, des résines polyvinyliques, des fluoropolymères, de la cire, des sels de métaux polyvalents d'acide amidosulfonique, des acides aminés acylés, et des mélanges de ceux-ci.

11. Pigment composite selon l'une quelconque des revendications 1 à 10, dans lequel le rapport pondéral de la (des) petite(s) particule(s) au(x) filtre(s) UV solide(s) inorganique(s) et/ou au(x) pigment(s) colorant(s) est de 50/50 à 90/10, de préférence de 50/50 à 80/20, et plus préférablement de 50/50 à 70/30.

12. Procédé de préparation d'un pigment composite, comprenant une étape consistant à soumettre :

    au moins une petite particule ayant une taille moyenne de particule supérieure à 100 nm et inférieure à 1 $\mu$m, de préférence inférieure à 600 nm, et plus préférablement inférieure à 400 nm ;
    au moins une particule de filtre UV solide inorganique et/ou au moins un pigment colorant ; et
    facultativement au moins un filtre UV additionnel,
    à un procédé de fusion mécanochimique.

**13.** Composition de pigment composite, comprenant :

au moins un pigment composite selon l'une quelconque des revendications 1 à 12 ; et
au moins une grande particule ayant une taille moyenne de particule de 2 μm ou plus, de préférence de 3 μm ou plus, plus préférablement de 4 μm ou plus, et encore plus préférablement de 5 μm ou plus,
dans lequel la surface de la grande particule est facultativement au moins en partie recouverte d'au moins une couche comprenant au moins une particule de filtre UV solide inorganique et/ou au moins un pigment colorant.

**14.** Composition de pigment composite selon la revendication 13, dans lequel la grande particule comprend au moins un matériau inorganique et/ou au moins un matériau organique, de préférence au moins un matériau organique.

**15.** Composition de pigment composite selon la revendication 13 ou 14, dans lequel le rapport pondéral de la (des) petite(s) particule(s) à la (aux) grande(s) particule(s) est de 10/90 à 90/10, de préférence de 20/80 à 80/20, et plus préférablement de 30/70 à 70/30.

**16.** Composition de pigment composite selon l'une quelconque des revendications 13 à 15, dans lequel le rapport pondéral de la (des) petite(s) particule(s)/la (les) grande(s) particule(s)/la (les) particule(s) de filtre UV solide inorganique et/ou le(s) pigment(s) colorant(s) est de 20/50/30 à 50/20/30, de préférence de 35/15/50 à 15/35/50, et plus préférablement de 10/20/70 à 20/10/70.

**17.** Procédé de préparation d'une composition de pigment composite, comprenant une étape consistant à soumettre :

au moins une petite particule ayant une taille moyenne de particule supérieure à 100 nm et inférieure à 1 μm, de préférence inférieure à 600 nm, et plus préférablement inférieure à 400 nm ;
au moins une particule de filtre UV solide inorganique et/ou au moins un pigment colorant ;
au moins une grande particule ayant une taille moyenne de particule de 2 μm ou plus, de préférence de 3 μm ou plus, plus préférablement de 4 μm ou plus, et encore plus préférablement de 5 μm ou plus ; et
facultativement au moins un filtre UV additionnel,
à un procédé de fusion mécanochimique.

**18.** Composition cosmétique, en particulier sous la forme d'un liquide ou d'une composition cosmétique en poudre, comprenant un pigment composite selon l'une quelconque des revendications 1 à 11 ou une composition de pigment composite selon l'une quelconque des revendications 13 à 16.

FIG. 1

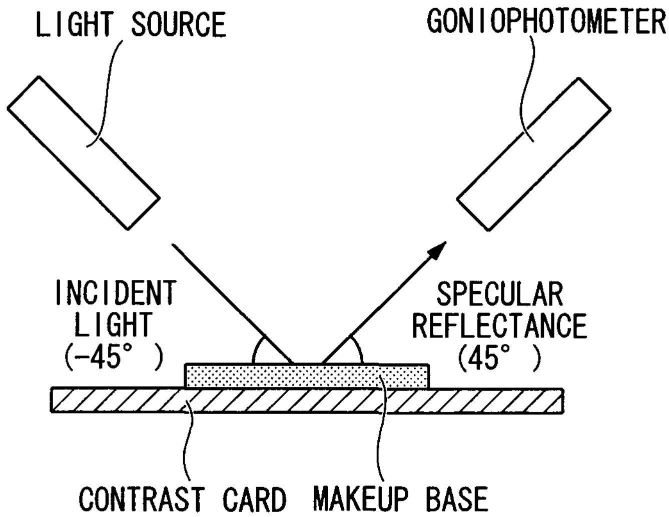
LIGHT SOURCE
GONIOPHOTOMETER
INCIDENT LIGHT (-45°)
SPECULAR REFLECTANCE (45°)
CONTRAST CARD
MAKEUP BASE

FIG. 2

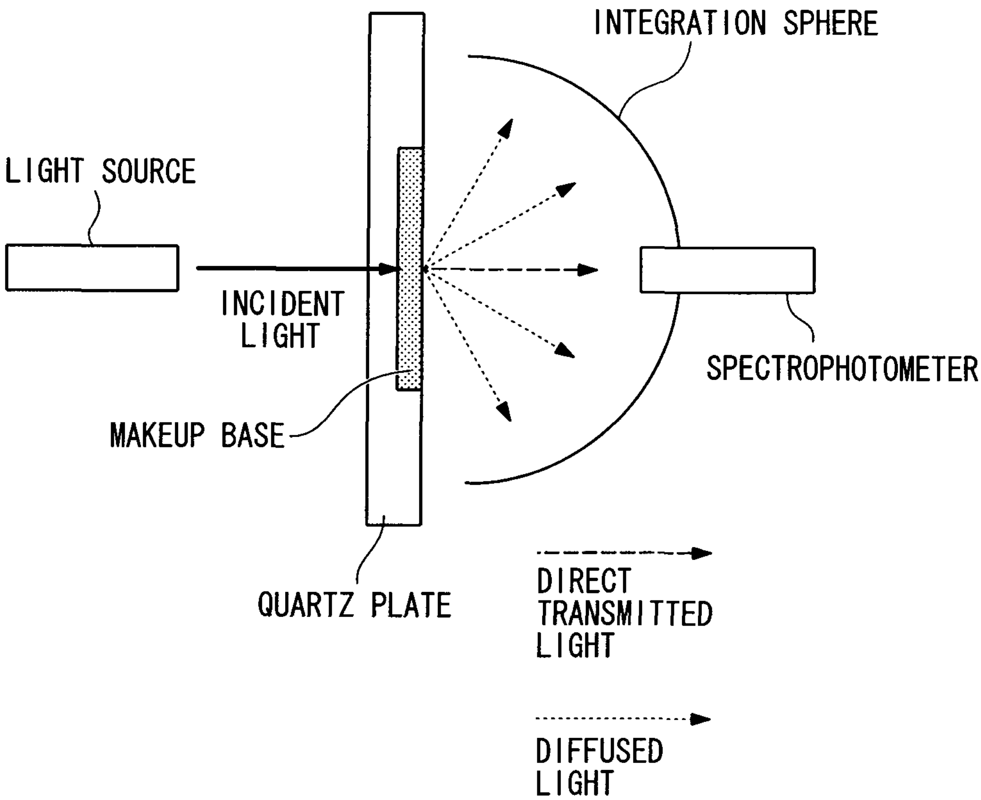
INTEGRATION SPHERE
LIGHT SOURCE
INCIDENT LIGHT
SPECTROPHOTOMETER
MAKEUP BASE
QUARTZ PLATE
DIRECT TRANSMITTED LIGHT
DIFFUSED LIGHT

FIG. 3

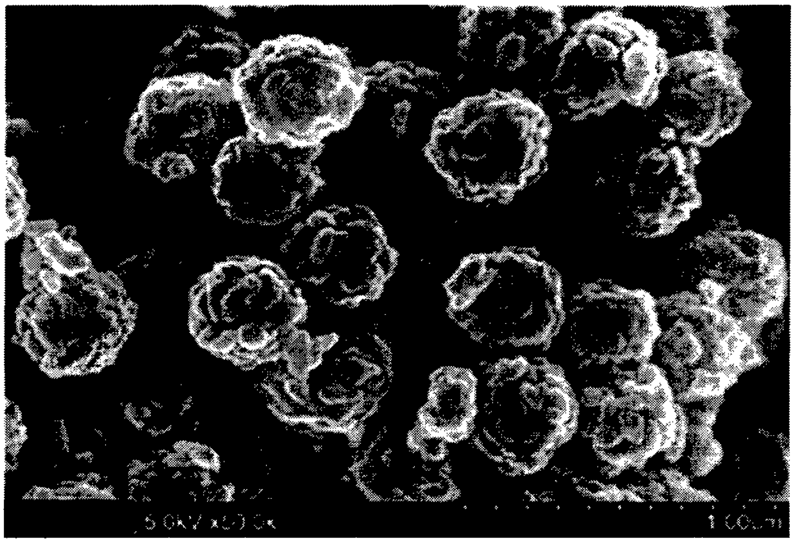

FIG. 4

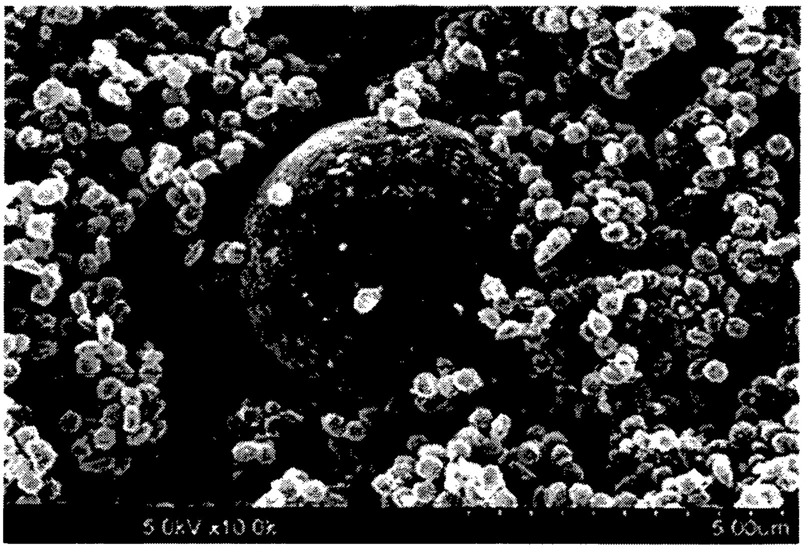

FIG. 5

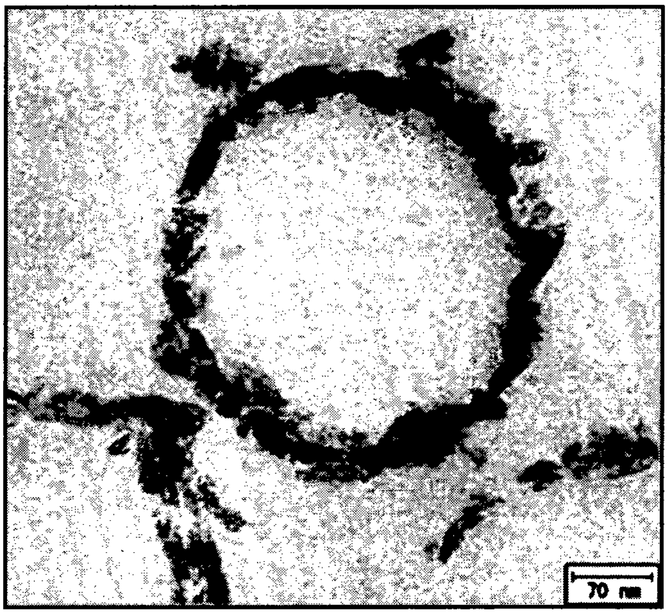


FIG. 6

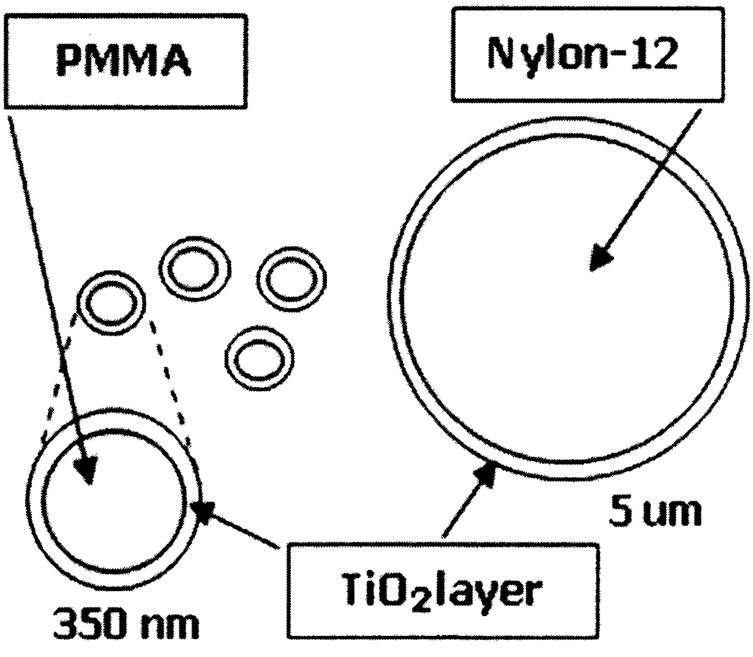

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007254429 A
- US 4617390 A
- US 6225467 B
- WO 2004085412 A
- WO 06035000 A
- WO 06034982 A
- WO 06034991 A
- WO 06035007 A
- WO 2006034992 A
- WO 2006034985 A
- US 5240975 A
- EP 669323 A
- US 2463264 A
- US 5237071 A
- US 5166355 A
- GB 2303549 A
- DE 19726184
- EP 893119 A
- WO 9304665 A
- DE 19855649
- EP 0293795 A
- JP 2295912 A
- EP 895779 A
- EP 524109 A
- WO 9910318 A
- WO 9932077 A
- WO 9922707 A
- WO 2004105736 A
- FR 2466492
- WO 9735842 A
- EP 903342 A
- WO 9725970 A
- FR 2840806
- FR 2651126
- EP 0425324 B
- US 5236986 A
- US 5412004 A
- US 5837793 A
- US 5811487 A
- WO 2004024798 A
- WO 9206778 A
- DE 19943681
- DE 19943668
- DE 4227391
- DE 19608117
- JP 11060437 A
- JP 8311003 A
- EP 0697244 A
- EP O697245 A
- EP 0708079 A
- DE 19622612
- JP 10017593 A
- WO 03024412 A
- US 5863886 A
- WO 9625388 A
- WO 9614926 A
- WO 9616930 A
- WO 9625384 A
- WO 9740124 A
- WO 9731890 A
- DE 19750246
- DE 19750245
- DE 19631225
- DE 19647060
- EP 1069142 A
- FR 2315991
- FR 2416008
- EP 295886 A
- US 4077441 A
- US 4850517 A


### Non-patent literature cited in the description


- *Cosmetics & Toiletries,* February 1990, vol. 105, 53-64
- Symmetrical Triazine Derivatives. IP.COM Journal. IP.COM INC, 20 September 2004
- **MILTON J. ROSEN.** Gemini Surfactants, Properties of surfactant molecules with two hydrophilic groups and two hydrophobic groups. *Cosmetics & Toiletries magazine,* December 1998, vol. 113, 49-55
- **MILTON J. ROSEN.** Recent Developments in Gemini Surfactants. *Allured's Cosmetics & Toiletries magazine,* July 2001, vol. 116 (7), 67-70
- **BANGHAM ; STANDISH ; WATKINS.** *J. Mol. Biol.,* 1965, vol. 13, 238
- **B.L. DIFFEY.** *J. Soc. Cosmet. Chem.,* 1989, vol. 40, 127-133